(19) Europäisches Patentamt
European Patent Office
Office européen des brevets

(11) **EP 4 650 704 A1**

(12) **EUROPEAN PATENT APPLICATION**
published in accordance with Art. 153(4) EPC

(43) Date of publication:
**19.11.2025 Bulletin 2025/47**

(21) Application number: **23955153.4**

(22) Date of filing: **12.10.2023**

(51) International Patent Classification (IPC):
***G01B 7/16*** (2006.01)

(52) Cooperative Patent Classification (CPC):
**A41D 19/00; A61B 5/00; A61B 5/11; G01B 7/16;
G06F 3/01; G06V 40/20**

(86) International application number:
**PCT/CN2023/124295**

(87) International publication number:
**WO 2025/076773 (17.04.2025 Gazette 2025/16)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Shenzhen Shokz Co., Ltd.
Shenzhen, Guangdong 518108 (CN)**

(72) Inventors:
• **DENG, Wenjun**
  **Shenzhen, Guangdong 518108 (CN)**

• **HUANG, Yujia**
  **Shenzhen, Guangdong 518108 (CN)**
• **YUAN, Yongshuai**
  **Shenzhen, Guangdong 518108 (CN)**
• **LIANG, Xianrong**
  **Shenzhen, Guangdong 518108 (CN)**
• **ZHOU, Wenbing**
  **Shenzhen, Guangdong 518108 (CN)**

(74) Representative: **Wang, Bo
Panovision IP
Ebersberger Straße 3
85570 Markt Schwaben (DE)**

(54) **SENSOR**

(57) Disclosed herein is a sensor, including: a flexible substrate; a first sensing structure, a second sensing structure, and a processing circuit. The first sensing structure and the second sensing structure each include a multilayer structure arranged on a same side surface of the flexible substrate in a thickness direction. Each layer of the multilayer structure is stacked in the thickness direction. The processing circuit reads first parameters, each of which related to a resistance or a capacitance of the first sensing structure and the second sensing structure, respectively. The processing circuit determines a deformation in at least two dimensions of the flexible substrate based on the first parameters.

FIG. 1

## Description

### TECHNICAL FIELD

The present disclosure relates to the technical field of electronic components, and in particular to sensors.

### BACKGROUND

With the increasing maturity of augmented reality/virtual reality (AR/VR) technology and the emergence of the metaverse concept, intelligent electronic devices demand enhanced human-computer interaction capabilities. Flexible angle sensors can be readily integrated into wearable devices, such as smart garments and smart gloves, enabling precise recognition and replication of human motion. These flexible angle sensors constitute an important underlying technology for the metaverse and have consequently garnered significant research interest. Human body joints exhibit varying degrees of freedom of movement, imposing correspondingly distinct requirements on sensors. Joints with a single degree of freedom (e.g., elbow joints, knee joints) can achieve accurate motion capture using a single-axis bending sensor. Conversely, joints possessing multiple degrees of freedom (e.g., shoulder joints, hip joints, wrist joints, thumb carpometacarpal joints) necessitate sensors that satisfy more stringent requirements.

Therefore, how to improve the accuracy and convenience of the sensors in detecting the bending movement situation of multi degrees of freedom is an urgent technical problem to be solved in the field.

### SUMMARY

One of the embodiments of the present disclosure provides a sensor. The sensor includes: a flexible substrate, a first sensing structure, and a second sensing structure. The first sensing structure and the second sensing structure each include a multilayer structure arranged on a same side surface of the flexible substrate in a thickness direction, and each layer of the multilayer structure is stacked in the thickness direction. The sensor further includes a processing circuit. The processing circuit reads first parameters, each of which related to a resistance or a capacitance of the first sensing structure and the second sensing structure respectively, and determines a deformation in at least two dimensions of the flexible substrate based on the first parameters.

One of the embodiments of the present disclosure provides a smart glove. The smart glove includes: a glove body, a sensor, and a processor configured to receive and process data collected by the sensor. The sensor is located in any one or more regions of the glove body corresponding to finger joints, metacarpophalangeal joints, carpometacarpal joints, and wrist joints of a user.

One of the embodiments of the present disclosure further provides a smart garment. The smart garment includes: a garment body, a sensor; and a processor configured to receive and process data collected by the sensor. The sensor is located in any one or more regions of the garment body corresponding to shoulder joints, spine joints, hip joints, and ankle joints of a user.

Additional features may be partially described in the following explanation, and may become apparent to those skilled in the art by reference to the following and the accompanying drawings, or may be appreciated by the generation or operation of examples. Features of the present disclosure may be realized and obtained by practicing or using aspects of the methods, tools, and combinations set forth in the following detailed examples.

### BRIEF DESCRIPTION OF THE DRAWINGS

The present specification will be further illustrated by way of exemplary embodiments, which may be described in detail by way of the accompanying drawings. These embodiments are not limiting, and in these embodiments, the same numbering denotes the same structure, where:

FIG. 1 is a schematic diagram illustrating a sensor according to some embodiments of the present disclosure;

FIG. 2 is a schematic diagram illustrating a distribution of two sensing structures on a flexible substrate according to some embodiments of the present disclosure;

FIG. 3 is a schematic diagram illustrating a distribution of two sensing structures on a flexible substrate according to some further embodiments of the present disclosure;

FIG. 4A is a schematic diagram illustrating a distribution of four sensing structures on a flexible substrate according to some embodiments of the present disclosure;

FIG. 4B is a schematic diagram of a cross-sectional structure along a Y-axis view according to FIG. 4A;

FIG. 5A is a schematic diagram illustrating a structure of a sensor without a deformation according to some embodiments of the present disclosure;

FIG. 5B is a schematic diagram illustrating a deformation of a sensor after stretching or after compression along a long axis direction according to FIG. 5A;

FIG. 6 is a schematic diagram illustrating a side view of a sensor after a bending deformation around an axis parallel to a short axis direction according to FIG. 5A;

FIG. 7 is a schematic diagram illustrating a top view of a sensor after a bending deformation around an axis parallel to a thickness direction according to FIG. 5A;

FIG. 8 is a schematic diagram illustrating a flexible sensor with a wrinkle according to some embodiments of the present disclosure;

FIG. 9 is a schematic diagram illustrating a curve obtained from actual measurement of a sensor according to FIG. 4A;

FIG. 10 is a schematic diagram illustrating a cross-section of a sensor according to some embodiments of the present disclosure;

FIG. 11 is an equivalent circuit diagram of a sensor according to FIG. 10;

FIG. 12 is a schematic diagram illustrating a cross-section of a sensor according to yet other embodiments of the present disclosure;

FIG. 13 is a schematic diagram illustrating a cross-section of a sensor provided with a connecting member according to FIG. 12;

FIG. 14 is a schematic diagram illustrating a cross-section of a sensor according to some other embodiments of the present disclosure;

FIG. 15 is a schematic diagram illustrating a cross-section of a sensor according to some further embodiments of the present disclosure;

FIG. 16 is a first schematic diagram illustrating a cross-section of a sensor according to some embodiments of the present disclosure;

FIG. 17 is a second schematic diagram illustrating a cross-section of a sensor according to some embodiments of the present disclosure;

FIG. 18 is a third schematic diagram illustrating a cross-section of a sensor according to some embodiments of the present disclosure;

FIG. 19 is a fourth schematic diagram illustrating a cross-section of a sensor according to some embodiments of the present disclosure;

FIG. 20 is a fifth schematic diagram illustrating a cross-section of a sensor according to some embodiments of the present disclosure;

FIG. 21A is a schematic diagram illustrating a structure of a smart glove according to some embodiments of the present disclosure;

FIG. 21B is a schematic diagram illustrating joints of a user's hand according to some embodiments of the present disclosure;

FIG. 22A is a front schematic diagram illustrating a smart garment according to some embodiments of the present disclosure; and

FIG. 22B is a rear schematic diagram illustrating a smart garment according to some embodiments of the present disclosure.

## DETAILED DESCRIPTION

In order to more clearly illustrate the technical solutions of the embodiments of the present disclosure, the accompanying drawings required to be used in the description of the embodiments will be briefly described below. Obviously, the accompanying drawings in the following description are only some examples or embodiments of the present disclosure, and it is possible for those skilled in the art to apply the present disclosure to other similar scenarios according to these drawings without creative labor. It should be understood that these exemplary embodiments are given only to enable those of ordinary skill in the art to better understand and thus realize the present disclosure, and are not intended to limit the scope of the present disclosure in any way. Unless obviously obtained from the context or the context illustrates otherwise, the same numeral in the drawings refers to the same structure or operation.

As shown in the present disclosure and the claims, unless the context clearly suggests an exception, the terms "a," "an," "one," and/or "the" do not refer specifically to the singular, but may also include the plural. Generally speaking, the terms "comprising," and "including" only indicate the inclusion of explicitly identified steps and elements, and these steps and elements do not constitute an exhaustive list. Methods or devices may also contain other steps or elements. The term "based on" is "based at least in part on." The term "one embodiment" means "at least one embodiment"; the term "another embodiment" means "at least one other embodiment".

In the description of the present disclosure, it is to be understood that the terms "up," "down", etc. indicate an orientation or positional relationship based on the orientation shown in the accompanying drawings. These terms are used solely for the purpose of facilitating the description of the present disclosure and simplifying the explanation, and are not intended to indicate or imply that the referenced devices or components must have a specific orientation or be constructed and operated in a particular orientation. Therefore, such terms should not be construed as limiting the scope of the present disclosure.

Additionally, the terms "first," and "second" are used for descriptive purposes only and are not to be construed as indicating or implying relative importance or implicitly specifying the quantity of the indicated technical features. Thus, the feature defined as "first," and "second" may expressly or implicitly include at least one such feature. In the description of the present disclosure, "plurality" means at least two, e.g., two, three, or the like, unless explicitly and specifically limited otherwise.

In the present disclosure, unless otherwise expressly specified and qualified, the terms "installation," "connected," "connection," and "fixed" etc., should be interpreted broadly. For example, a connection may be fixed or detachable, or it may be integrated; it may be a mechanical connection or an electrical connection; it may be directly connected or indirectly connected through an intermediary medium; it may refer to the internal communication between two elements or the interaction between two elements, unless explicitly stated otherwise. To one of ordinary skill in the art, the specific meanings of the above terms in the present disclosure may be understood on a case-by-case basis.

Embodiments of the present disclosure provide a sensor. The sensor is susceptible to a deformation when subjected to an external force and converts the deformation into an electrical signal. When the sensor is disposed on a smart wearable device (e.g., a motion capture suit, a myoelectric suit, a motion capture glove, etc.), the electrical signal generated by the sensor may reflect a direction, a magnitude, etc., of a deformation at a corresponding position. In some scenarios, a position where the sensor is located may undergo a complex deformation. For example, when the sensor is worn near shoulder joints, hip joints, or finger root joints of a user, the joints have at least two different degrees of freedom, which in turn cause the sensor to undergo a deformation in multi-dimensions (e.g., bending, stretching, compression, etc., along different directions). At this time, by providing a plurality of sensing structures on the sensor in a specific manner and integrating electrical signals generated by the plurality of sensing structures, it is possible to achieve sensing of deformation situation in the multi-dimensions at the position where the sensor is located, thereby enabling precise recognition and reconstruction of human movements.

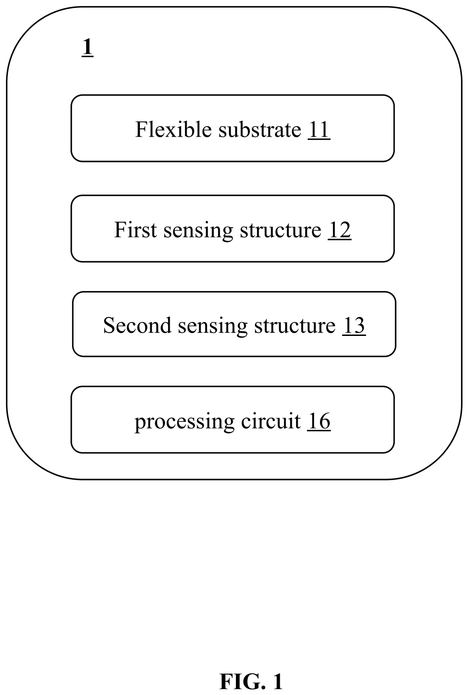

FIG. 1 is a schematic diagram illustrating a sensor according to some embodiments of the present disclosure.

As shown in FIG. 1, in some embodiments, the sensor 1 may include a flexible substrate 11, a first sensing structure 12, and a second sensing structure 13. The first sensing structure 12 and the second sensing structure 13 each includes a multilayer structure arranged on a same side surface of the flexible substrate 11 in a thickness direction. Each layer of the multilayer structure is stacked in the thickness direction. In some embodiments, the sensor 1 includes a processing circuit 16. The processing circuit 16 reads first parameters, each of which is related to a resistance or a capacitance of the first sensing structure 12 and the second sensing structure 13, respectively. The processing circuit 16 determines a deformation in at least two dimensions of the flexible substrate 11 based on the first parameters. The multilayer structure refers to a structure including a stack of layers. In some embodiments, the multilayer structure includes a second conductive layer, an intermediate layer, and a first conductive layer. In some embodiments, the multilayer structure includes the second conductive layer, a second intermediate layer, a third conductive layer, a first intermediate layer, and the first conductive layer. Specific layers regarding the multilayer structure may be found below.

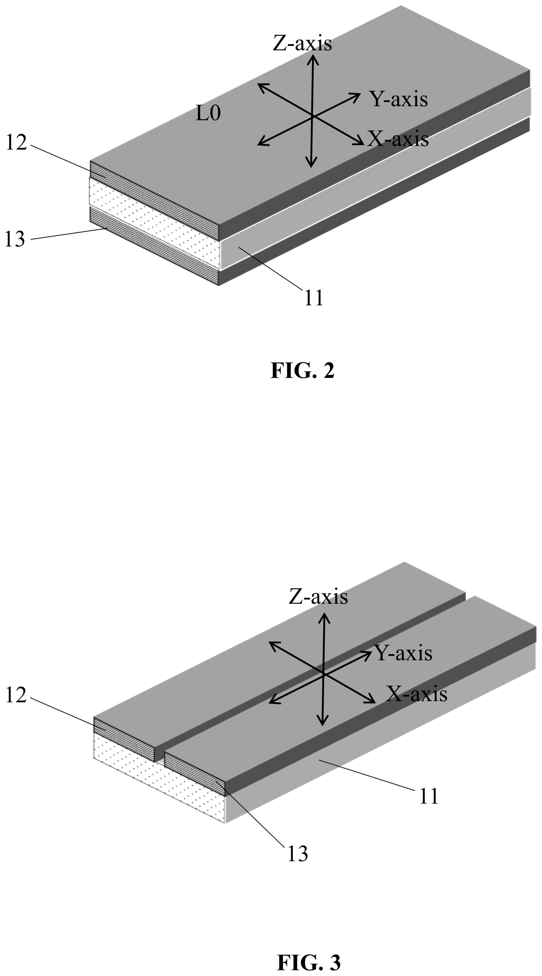

The flexible substrate 11 has flexible qualities and is susceptible to a deformation (e.g., a bending deformation) when subjected to an external force. In some embodiments, the flexible substrate may have a flattened structure to be easily set up in a smart wearable device and to easily fit into a joint of the human body. At this time, the flexible substrate 11 has the thickness direction, which may be a Z-axis direction as shown in FIG. 2. To sense the deformation of the flexible substrate 11, the flexible substrate 11 may be provided with sensing structures that are capable of converting a physical deformation into an electrical signal. For example, the flexible substrate 11 may serve as a support substrate for the first sensing structure 12 and the second sensing structure 13.

The sensing structures (e.g., the first sensing structure 12 and the second sensing structure 13) refer to sensing structures configured to measure the bending deformation of the flexible substrate 11 (or the sensor 1). The first sensing structure 12 and the second sensing structure 13 each includes a plurality of layered structures. Each layer of the multilayer structure being stacked in the thickness direction may be understood as follows: each layer of the plurality of layered structures of the first sensing structure 12 and the second sensing structure 13 is arrayed in the thickness direction of the flexible substrate 11 and stacked together. In some embodiments, either the first sensing structure 12 or the second sensing structure 13 may be any one of a capacitive sensing structure, a resistive sensing structure, and a composite capacitive-resistive sensing structure. The composite capacitive-resistive sensing structure refers to a sensing structure including a capacitance and a resistance, and whose output parameters (e.g., a voltage, a resistance) are subjected to a composite effect of the capacitance and the resistance. It should be noted that the capacitive sensing structure or the composite capacitive-resistive sensing structure includes an element or a structure having capacitive properties configured to store electrical energy. Exemplary elements or structures having capacitive properties include two conductors (electrodes) and a dielectric layer disposed between the two conductors (the electrodes). In some embodiments, the first sensing structure 12 and the second sensing structure 13 are distributed on both sides of the flexible substrate 11 in the thickness direction. In some embodiments, the first sensing structure 12 and the second sensing structure 13 may be distributed on a same side surface of the flexible substrate 11 in the thickness direction, as described in detail in FIG. 2 and FIG. 3 and the related descriptions.

In some embodiments, parameters of the sensor 1 may be read by the processing circuit 16. A specific type of the parameters that is read by the processing circuit 16 is related to a structure of the processing circuit 16, connectivity of the processing circuit 16 to the sensor 1, a structure of the sensor 1, or the like, as described below. In some embodiments, the processing circuit 16 may include a signal output device. The signal output device may output an electrical signal (e.g., a voltage signal) to the sensor 1 to enable the processing circuit 16 to correspondingly read the parameters fed back from the sensor 1. In some embodiments, the sensing structure may be the resistive sensing structure. At this time, the signal output device may output a direct current (DC) signal to the sensor 1 to enable the processing circuit 16 to read changes in the resistance of the resistive sensing structure in response to the deformation. In other embodiments, the sensing structure may be the capacitive sensing structure or the composite capacitive-resistive sensing structure. The signal output device may output an alternating current (AC) signal to the sensor 1 to enable the processing circuit 16 to read changes in the capacitance of the capacitive sensing structure or the composite capacitive-resistive sensing structure in response to the deformation. Merely by way of example, the signal output device may include a voltage output device. The voltage output device may output a voltage (DC voltage or AC voltage) to the sensor 1. The processing circuit 16 may correspondingly read a voltage fed back to the sensor 1 based on the voltage output by the voltage output device to the sensor 1. In some embodiments, the voltage output device may output a signal of a square wave, a triangle wave, a sine wave, a pulse wave, or the like, to the sensor 1. In some embodiments, the processing circuit 16 may read detection parameters of the first sensing structure 12 and the second sensing structure 13, respectively. The detection parameters include a parameter of the first sensing structure 12 related to the resistance or the capacitance, a parameter of the first sensing structure 12 related to the resistance and the capacitance, a parameter of the second sensing structure 13 related to the capacitance or the resistance, or a parameter of the second sensing structure 13 related to the resistance and the capacitance. Related descriptions of specific parameters included in the detection parameters may be found below.

When different sensing structures are arranged at different positions of the sensor 1, respectively, the sensing structures produce differentiated responses to the deformations in different dimensions of the flexible substrate 11. When the flexible substrate 11 produces deformations of a certain dimension, the detection parameters generated by the sensing structures have features corresponding to the deformations of the dimension. When the flexible substrate 11 produces deformations of another dimension, the detection parameters generated by the sensing structures may again have features corresponding to the deformations of the dimension. It should be understood that the detection parameters generated by the sensing structures have features that correspond to the dimensions of the deformations of the flexible substrate 11, and it is possible to recognize the deformations of the flexible substrate 11 on the basis of the detection parameters of the sensing structures. In some embodiments, the processing circuit 16 may determine the deformation of the flexible substrate 11 in the at least two dimensions by a specific algorithm based on the first parameters related to the resistance or the capacitance of the each sensing structure (e.g., the first sensing structure 12, the second sensing structure 13). For example, the processing circuit 16 may determine the deformation in the at least two dimensions of the flexible substrate 11 through machine learning models, mapping relationships, or functional relationships. A description of the deformation in the at least two dimensions may be found below.

The bending deformation of the sensor 1 (the flexible substrate 11) causes a physical shape of at least a portion of the sensing structures (e.g., the first sensing structure 12 and the second sensing structure 13) located on the flexible substrate 11 to change accordingly, which results in a change in the resistance or the capacitance corresponding to the at least a portion of the sensing structures. For example, bending of the sensor 1 causes a change in an area of the first sensing structure 12 and the second sensing structure 13, thereby changing the resistance of the first sensing structure 12 and the second sensing structure 13. The bending of the sensor 1 (e.g., a bending angle, a bending direction) can be sensed accurately by collecting the first parameters related to the resistance or the resistance of the first sensing structure 12 and the second sensing structure 13, and analyzing changes of the first parameters related to the resistance or the resistance of the first sensing structure 12 and the second sensing structure 13.

FIG. 2 is a schematic diagram illustrating a distribution of two sensing structures on a flexible substrate according to some embodiments of the present disclosure.

As shown in FIG. 2, in some embodiments, the first sensing structure 12 and the second sensing structure 13 are distributed on both sides of the flexible substrate 11 in the thickness direction. A projection of the first sensing structure 12 on the flexible substrate 11 at least partially overlaps with a projection of the second sensing structure 13 on the flexible substrate 11.

In some embodiments, the first sensing structure 12 and the second sensing structure 13 are distributed symmetrically along the flexible substrate 11 in the thickness direction. At this time, the projection of the first sensing structure 12 on the flexible substrate 11 and the projection of the second sensing structure 13 on the flexible substrate 11 completely overlap. When the first sensing structure 12 and the second sensing structure 13 are distributed symmetrically about the flexible substrate 11 in the thickness direction, the two sensing structures have a same response to a partial external disturbance (e.g., an overall stretching or compression of the sensor 1 along a certain direction). At this time, the processing circuit 16, based on first parameters related to the resistance or the capacitance of the two sensing structures, may exclude the influence of external interference on the sensor 1 through a differential processing algorithm, thereby improving sensitivity in determining a deformation of the flexible substrate 11 in at least two dimensions.

In some embodiments, the flexible substrate 11 includes a short axis direction and a long axis direction both perpendicular to the thickness direction. Deformations generated by the sensor 1 (the flexible substrate 11) shown in FIG. 2 in a plurality of dimensions at least include a bending deformation around an axis parallel to the short axis direction and a tensile or compressive deformation in the long axis direction. The short axis direction may be an X-axis direction in FIG. 2; and the long axis direction may be a Y-axis direction in FIG. 2.

As shown in FIG. 2, when the sensor 1 undergoes the bending deformation around an axis parallel to the short axis direction (e.g., the X-axis direction), the first sensing structure 12 and the second sensing structure 13 undergo a tensile deformation and a compressive deformation, respectively. For example, the first sensing structure 12 may be bent and elongated, and the second sensing structure 13 may be bent and compressed. At this time, the resistance (or the capacitance) of the first sensing structure 12 and the second sensing structure 13 changes in opposite directions. In this case, a difference between the resistance (or the capacitance) of the first sensing structure 12 and the second sensing structure 13 may reflect a bending direction and a bending degree of the sensor 1 around the axis parallel to the X-axis direction. And because the first sensing structure 12 and the second sensing structure 13 are stretched and compressed to substantially the same degree, a sum of the resistance (or the capacitance) may remain substantially constant.

When the sensor 1 undergoes the tensile or compressive deformation in the long axis direction, the first sensing structure 12 and the second sensing structure 13 may be stretched or compressed synchronously, and at this time, the resistance (or the capacitance) of the first sensing structure 12 and the second sensing structure 13 may change synchronously. At this time, the difference between the resistance (or the capacitance) of the first sensing structure 12 and the second sensing structure 13 remains essentially constant (or close to 0), while the sum of the resistance (or the capacitance) of the first sensing structure 12 and the second sensing structure 13 may reflect the degree of stretching or compression of the sensor 1 in the long axis direction.

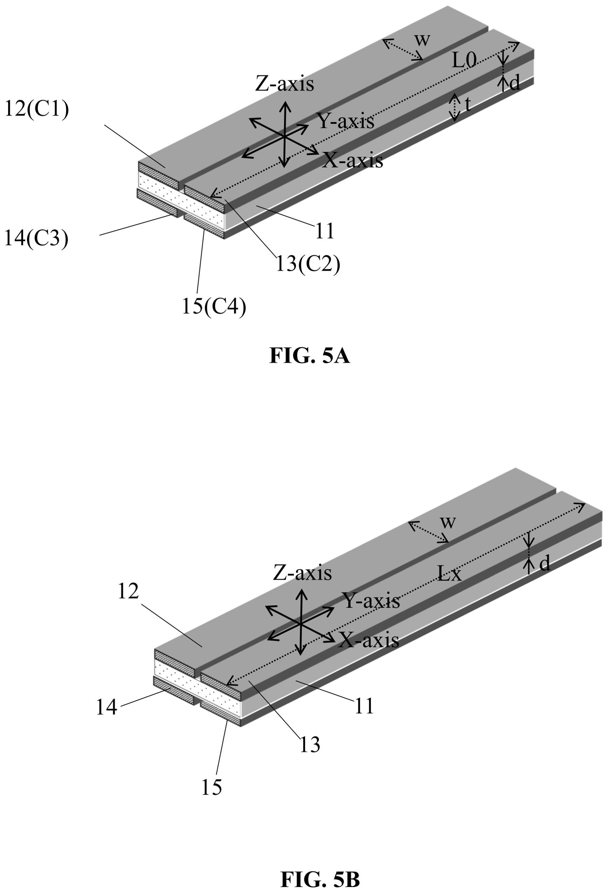

In some embodiments, by comparing the features of the resistance (or the capacitance) of the first sensing structure 12 and the second sensing structure 13 in the two dimensions, for example, the relationship between the sum (or the difference) of the resistance (or the capacitance) and the deformation of the flexible substrate 11, mutual interference between the deformations in the two dimensions can be avoided, and the deformations of the flexible substrate 11 in the two dimensions can be effectively distinguished. For example, when the sensor 1 is overall stretched or compressed, the first sensing structure 12 and the second sensing structure 13 are stretched or compressed synchronously. By treating the synchronous stretching or compression of the first sensing structure 12 and the second sensing structure 13 as a common-mode interference, differential processing of the signals (such as the resistance or the capacitance) may exclude such common-mode interference, which makes the sensor 1 insensitive to its own tensile or compressive deformation while remaining sensitive to the bending deformation around the axis parallel to the short axis direction, thereby enabling the sensor 1 to accurately detect the bending deformation in the dimension. That is, the difference between the resistance (or the capacitance) of the first sensing structure 12 and the second sensing structure 13 primarily reflects the bending deformation of the flexible substrate 11 around the axis parallel to the short axis direction. Similarly, the sum of the resistance (or the capacitance) of the first sensing structure 12 and the second sensing structure 13 primarily reflects the tensile or compressive deformation of the flexible substrate 11 in the long axis direction. A detection principle of the tensile or compressive deformation in the long axis direction is similar to a detection principle of the sensor 1 with four sensing structures. The specific description may be found in FIG. 5A to FIG. 7 and the related descriptions.

FIG. 3 is a schematic diagram illustrating a distribution of two sensing structures on a flexible substrate according to some further embodiments of the present disclosure.

As shown in FIG. 3, in some embodiments, the flexible substrate 11 includes a long axis direction perpendicular to a thickness direction. The first sensing structure 12 and the second sensing structure 13 are distributed on the same side surface of the flexible substrate 11 in the thickness direction. The first sensing structure 12 and the second sensing structure 13 are disposed side by side and both extend in the long axis direction of the flexible substrate 11.

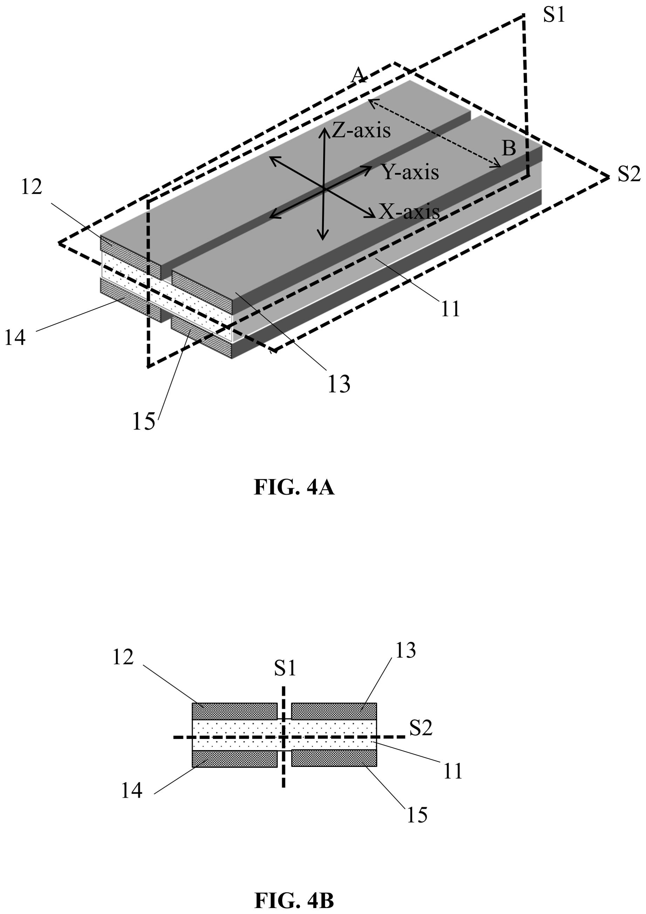

In some embodiments, the first sensing structure 12 and the second sensing structure 13 are symmetrically disposed along a mid-section parallel to a plane formed by the thickness direction and the long axis direction on the flexible substrate 11. When the first sensing structure 12 and the second sensing structure 13 are distributed symmetrically about the flexible substrate 11 in the mid-section, the two sensing structures have a same response to a partial external disturbance (e.g., an overall stretching or compression of the sensor 1 along a certain direction). At this time, the processing circuit 16, based on first parameters related to a resistance or a capacitance of the two sensing structures, may exclude the influence of external interference on the sensor 1 through a differential processing algorithm, thereby improving sensitivity in determining a deformation of the flexible substrate 11 in at least two dimensions. More details regarding the mid-section may be found in FIG. 4A and FIG. 4B and the related descriptions.

In some embodiments, a deformation in the at least two dimensions produced by the sensor 1 (the flexible substrate 11) shown in FIG. 3 includes a bending deformation around an axis parallel to the thickness direction and a tensile or compressive deformation in the long axis direction.

As shown in FIG. 3, when the sensor 1 undergoes the bending deformation around the axis parallel to the thickness direction (e.g., a Z-axis direction), the first sensing structure 12 and the second sensing structure 13 undergo a tensile deformation and a compressive deformation, respectively. For example, the first sensing structure 12 may be bent and elongated, and the second sensing structure 13 may be bent and compressed. At this time, the resistance (or the capacitance) of the first sensing structure 12 and the second sensing structure 13 changes in opposite directions. In this case, a difference between the resistance (or the capacitance) of the first sensing structure 12 and the second sensing structure 13 may reflect a bending direction and a bending degree of the sensor 1 around the axis parallel to the Z-axis direction. And because the first sensing structure 12 and the second sensing structure 13 are stretched and compressed to substantially the same degree, a sum of the resistance (or the capacitance) may remain substantially constant.

When the sensor 1 shown in FIG. 3 undergoes the tensile or compressive deformation in the long axis direction, the first sensing structure 12 and the second sensing structure 13 may be stretched or compressed synchronously, and at this time, the resistance (or the capacitance) of the first sensing structure 12 and the second sensing structure 13 may change synchronously. At this time, the difference between the resistance (or the capacitance) of the first sensing structure 12 and the second sensing structure 13 remains essentially constant (or close to 0), while the sum of the resistance (or the capacitance) of the first sensing structure 12 and the second sensing structure 13 may reflect the degree of stretching or compression of the sensor 1 in the long axis direction.

In some embodiments, by comparing the features of the resistance (or the capacitance) of the first sensing structure 12 and the second sensing structure 13 in the two dimensions (e.g., the relationship between the sum (or the difference) of the resistance (or the capacitance) and the deformation of the flexible substrate 11), mutual interference between the deformations in the two dimensions can be avoided, and the deformations of the flexible substrate 11 in the two dimensions can be effectively distinguished. For example, when the sensor 1 is overall stretched or compressed, the first sensing structure 12 and the second sensing structure 13 may be stretched or compressed synchronously. By treating the synchronous stretching or compression of the first sensing structure 12 and the second sensing structure 13 as a common-mode interference, differential processing of the signals (such as the resistance or the capacitance) can exclude such common-mode interference, which makes the sensor 1 insensitive to its own tensile or compressive deformation while remaining sensitive to the bending deformation around the axis parallel to the thickness direction, thereby enabling the sensor 1 to accurately detect the bending deformation in the dimension. That is, the difference between the resistance (or the capacitance) of the first sensing structure 12 and the second sensing structure 13 primarily reflects the bending deformation of the flexible substrate 11 around the axis parallel to the thickness direction. Similarly, the sum of the resistance (or the capacitance) of the first sensing structure 12 and the second sensing structure 13 primarily reflects the tensile or compressive deformation of the flexible substrate 11 in the long axis direction. The detection principle of the tensile or compressive deformation in the long axis direction is similar to the detection principle of the sensor 1 with four sensing structures. The specific description may be found in FIG. 5A to FIG. 7 and the related descriptions.

In some embodiments, the sensor 1, as shown in FIG. 2 and FIG. 3, includes a shielding layer. The shielding layer is provided around a periphery of the flexible substrate 11 to shield the flexible substrate 11 from the outside world. For example, the first sensing structure 12 and the second sensing structure 13 are connected through a connecting member having an electrical conductivity. The connecting member is connected to electrically conductive electrodes on the first sensing structure 12 and the second sensing structure 13 to form the shielding layer that is provided around the periphery of the flexible substrate 11. Descriptions of the shielding layer and the connecting member may be found below.

FIG. 4A is a schematic diagram illustrating a distribution of four sensing structures on a flexible substrate according to some embodiments of the present disclosure. FIG. 4B is a schematic diagram of a cross-sectional structure along a Y-axis view according to FIG. 4A.

As shown in FIG. 4A and FIG. 4B, in some embodiments, the sensor 1 further includes a third sensing structure 14 and a fourth sensing structure 15 compared to FIG. 3. The third sensing structure 14 and the fourth sensing structure 15, each of which include a multilayer structure arranged on a same side surface of the flexible substrate 11 in a thickness direction, and each layer of the multilayer structure is stacked in the thickness direction. The flexible substrate 11 includes a long axis direction and a short axis direction both perpendicular to the thickness direction. The first sensing structure 12 and the second sensing structure 13 are distributed side by side on one side surface of the flexible substrate 11 in the thickness direction and both extend in the long axis direction. And the third sensing structure 14 and the fourth sensing structure 15 are distributed side by side on the other side surface of the flexible substrate 11 in the thickness direction, and both extend in the long axis direction.

In some embodiments, the processing circuit 16 reads first parameters, each of which is related to a resistance or a capacitance of the first sensing structure 12 and the second sensing structure 13. The processing circuit 16 reads second parameters, each of which is related to a resistance or a capacitance of the third sensing structure 14 and the fourth sensing structure 15, respectively. The processing circuit 16 determines, based on the first parameters and the second parameters, a deformation in at least two dimensions of the flexible substrate 11.

In some embodiments, the deformation in the at least two dimensions produced by the sensor 1 (the flexible substrate 11) shown in FIG. 4A includes a bending deformation around an axis parallel to the thickness direction, a bending deformation around an axis parallel to the short axis direction, and a tensile or compressive deformation in the long axis direction of the flexible substrate 11. For illustrative purposes, the parameters related to the resistance or the capacitance of each sensing structure under the deformation in different dimensions are described below in conjunction with FIG. 5A, FIG. 5B, FIG. 6, and FIG. 7, respectively.

In some embodiments, the parameters related to the capacitance include: C1, C2, C3 , C4, Ctotal, dCx, dCz. Ctotal = C1 + C2 + C3 + C4. The dCx that is obtained by compound difference operation is represented by dCx = (C1 + C2) - (C3 + C4). The dCz that is obtained by compound difference operation is represented by dCz = (C1 + C3) - (C2 + C4). C1, C2, C, and C denote the capacitance of the first sensing structure 12, the second sensing structure 13, the third sensing structure 14, and the fourth sensing structure 15, respectively. In some embodiments, the parameters related to the resistance include: R1, R2, R, and R. R1, R2, R, and R denote the resistance of the first sensing structure 12, the second sensing structure 13, the third sensing structure 14, and the fourth sensing structure 15, respectively.

In some embodiments, the first sensing structure 12 and the second sensing structure 13 are distributed symmetrically about a first mid-section S1, and the third sensing structure 14 and the fourth sensing structure 15 are distributed symmetrically about the first mid-section S1. The first mid-section S1 represents a mid-section in the flexible substrate 11 parallel to a plane formed by the thickness direction and the long axis direction. The first sensing structure 12 and the third sensing structure 14 are distributed symmetrically about a second mid-section S2 of the flexible substrate 11, and the second sensing structure 13 and the fourth sensing structure 15 are distributed symmetrically about the second mid-section S2. The second mid-section S2 represents a mid-section in the flexible substrate 11 parallel to a plane formed by the long axis direction and the short axis direction.

By providing symmetrical four sensing structures on the sensor 1 and performing a compound difference operation, a common-mode interference may be effectively excluded in a process of recognizing the bending deformation of the sensor. Merely by way of example, the first sensing structure 12, the second sensing structure 13, the third sensing structure 14, and the fourth sensing structure 15 are synchronized to stretch or compress when the sensor 1 is overall stretched or compressed. By treating the stretching or compression of the four sensing structures as the common-mode interference, composite differential processing of the signals from the four sensing structures ensures that dCx and dCz remain unchanged, effectively eliminating the common-mode interference, which makes the flexible sensor 1 insensitive to its own tensile or compressive deformation while remaining sensitive to the bending deformation, thereby enhancing the accuracy of sensor 1.

FIG. 5A is a schematic diagram illustrating a structure of a sensor without a deformation according to some embodiments of the present disclosure. FIG. 5B is a schematic diagram illustrating a deformation of a sensor after stretching or after compression along a long axis direction according to FIG. 5A.

In some embodiments, when the first sensing structure 12, the second sensing structure 13, the third sensing structure 14, and the fourth sensing structure 15 are capacitive sensing structures, the sensor 1 is capable of recognizing, based on the parameters related to the capacitance, the tensile or compressive deformation of the flexible substrate 11 in the long axis direction.

In some embodiments, as shown in FIG. 5A, when the sensor 1 is not deformed, the capacitance of the four sensing structures may be obtained from the following equation (1). For illustrative purposes only, at this time, the four sensing structures of the sensor 1 are regarded as identical and symmetrical about the flexible substrate 11 (e.g., the first mid-section S1 and the second mid-section S2) in both vertical and horizontal directions.

$$C1 = C2 = C3 = C4 = \varepsilon_0 \varepsilon \frac{A}{d} = \varepsilon_0 \varepsilon \frac{L0w}{d} \ (1),$$

where, $\varepsilon_0$ denotes a vacuum dielectric constant; $\varepsilon$ denotes a relative dielectric constant of each of the four sensing structures (e.g., an intermediate layer, see below); d denotes a thickness of a dielectric layer of each of the four sensing structures; A denotes an area of a plane formed by each of the four sensing structures in both the long axis direction and the short axis direction; L0 denotes an initial length of each of the four sensing structures in the long axis direction of the flexible substrate 11; and w denotes a width of each of the four sensing structures in the short axis direction of the flexible substrate 11.

In some embodiments, as shown in FIG. 5B, after the sensor 1 is stretched or compressed in the long axis direction (e.g., a Y-axis direction), the initial length L0 of each of the four sensing structures is stretched or compressed to Lx. Considering that the thickness variations of the four sensing structures are minimal, for the sake of simplicity, it is approximately assumed that the thickness of each of the four sensing structures remains unchanged. Therefore, according to equation (1), the capacitance of the four sensing structures may be varied as equation (2):

$$C1 = C2 = C3 = C4 = \varepsilon_0\varepsilon\frac{Lxw}{d} \quad (2).$$

At this time, Ctotal, dCx, and dCz are determined by the following equations (3), (4) and (5), respectively.

$$Ctotal = C1 + C2 + C3 + C4 = 4\varepsilon_0\varepsilon\frac{Lxw}{d} \quad (3),$$

$$dCx = (C1 + C2) - (C3 + C4) = 0 \quad (4),$$

$$dCz = (C1 + C3) - (C2 + C4) = 0 \quad (5).$$

When the sensor 1 undergoes the tensile or compressive deformation in the long axis direction, the four sensing structures are stretched or compressed synchronously, and at this time, the capacitance of the four sensing structures undergoes synchronized changes. In this case, a sum of the capacitance of the four sensing structures may reflect a degree of stretching or compression of the sensor 1 (the flexible substrate 11) in the long axis direction. According to equations (2) and (3), Ctotal is only related to the changed length Lx of the sensing structure, and Lx represents the tensile or compressive deformation of the sensor 1 in the long axis direction. Therefore, Ctotal may be configured to reflect the tensile or compressive deformation of the sensor 1 (the flexible substrate 11) in the long axis direction.

According to equation (4), dCx reflects a difference between a sum of the capacitance C1 of the first sensing structure 12 and the capacitance C2 of the second sensing structure 13 and a sum of the capacitance C3 of the third sensing structure 14 and the capacitance C4 of the fourth sensing structure 15. Referring to a structural distribution of the four sensing structures in FIG. 5B, it may be seen that the sum (C1+C2) of the capacitance C1 of the first sensing structure 12 and the capacitance C2 of the second sensing structure 13 reflects a bending deformation of an upper side surface of the flexible substrate 11 in the thickness direction, the sum (C3+C4) of the capacitance C3 of the third sensing structure 14 and the capacitance C4 of the fourth sensing structure 15 reflects a bending deformation of a lower side surface of the flexible substrate 11 in the thickness direction. Therefore, dCx is used to reflect the difference in the bending deformations of the upper and lower sides (e.g., the upper and lower sides in the thickness direction) of the flexible substrate 11 when the flexible substrate 11 is deformed, and is related to the bending deformation around the axis parallel to the short axis direction (e.g., an X-axis direction), whereas the deformation of the stretching or the compression in the long axis direction (e.g., a Y-axis direction) may not result in the difference in the deformation between the upper and lower sides of the flexible substrate 11, so dCx is a constant 0.

Similarly, according to equation (5), dCz is used to reflect a difference in the bending deformations produced in the short axis direction of the two side surfaces of the flexible substrate 11 during the deformation. dCz is only related to a bending deformation around the axis parallel to the thickness direction (e.g., the Z-axis direction), and a deformation in tension or compression in the long axis direction (e.g., the Y-axis direction) does not cause a difference in the deformation of the two side surfaces of the flexible substrate 11 in the short axis direction, so dCz is also a constant 0.

In some embodiments, output parameters Ctotal of the sensor 1 stretched or compressed in the Y-axis varies linearly in proportion to a tensile length or a compression length Lx, exhibiting extremely high linearity. And dCz and dCx are both 0, which are independent of the tensile length or the compression length Lx. Thus, the sensor 1 is capable of recognizing, based on the parameters Ctotal related to the capacitance, the tensile or compressive deformation of the flexible substrate in the long axis direction.

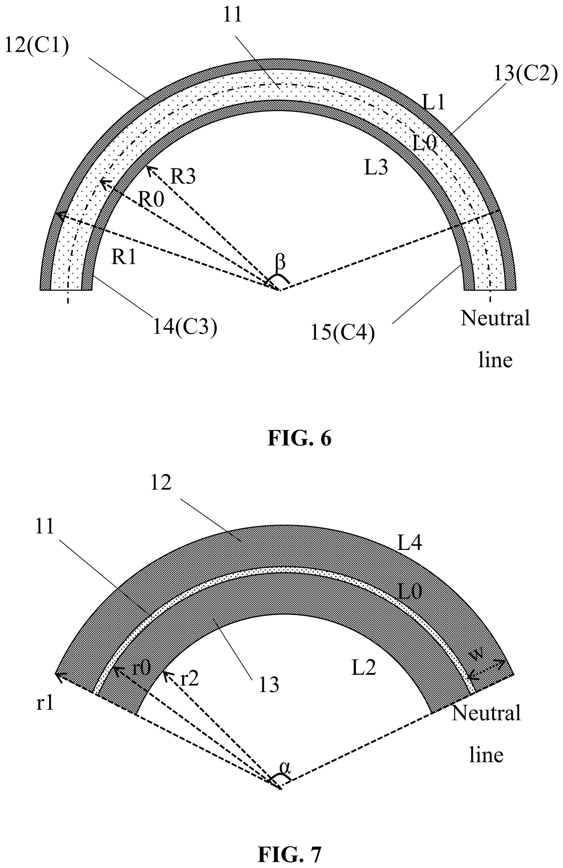

FIG. 6 is a schematic diagram illustrating a side view of a sensor after a bending deformation around an axis parallel to a short axis direction according to FIG. 5A.

In some embodiments, when the first sensing structure 12, the second sensing structure 13, the third sensing structure 14, and the fourth sensing structure 15 are all capacitive sensing structures, the sensor 1 is capable of recognizing, based on the parameters dCx related to the capacitance, the bending deformation of the flexible substrate 11 around the axis parallel to the short axis direction.

As shown in FIG. 5A and FIG. 6, when the sensor 1 undergoes the bending deformation around the axis parallel to the short axis direction (e.g., the X-axis direction), the sensor 1 after the bending deformation is approximated to be a circular arc for ease of illustration. The first sensing structure 12 and the second sensing structure 13 are bent and stretched to L1, and the third sensing structure 14 and the fourth sensing structure 15 are bent and compressed to L3. Considering that a thickness of each of the four sensing structures varies very little, it is approximated that the thickness of each of the four sensing structures remains constant for ease of illustration. Then, according to equation (1), the capacitance C1 of the first sensing structure 12 and the capacitance C2 of the second sensing structure 13 change as equation (6) below, and the capacitance C3 of the third sensing structure 14 and the capacitance C4 of the fourth sensing structure 15 change as equation (7) below.

$$C1 = C2 = \varepsilon_0\varepsilon\frac{L1w}{d} = \varepsilon_0\varepsilon\frac{R1\beta w}{d} = \varepsilon_0\varepsilon\frac{\left(R0+\frac{t}{2}\right)\beta w}{d} \quad (6),$$

$$C3 = C4 = \varepsilon_0\varepsilon\frac{L3w}{d} = \varepsilon_0\varepsilon\frac{R3\beta w}{d} = \varepsilon_0\varepsilon\frac{\left(R0-\frac{t}{2}\right)\beta w}{d} \quad (7).$$

At this time, Ctotal, dCx, and dCz are determined by the following equations (8), (9) and (10).

$$Ctotal = C1 + C2 + C3 + C4 = 4\varepsilon_0\varepsilon\frac{L0w}{d} \quad (8),$$

$$dCx = (C1 + C2) - (C3 + C4) = \frac{2\varepsilon_0\varepsilon wt}{d}\beta \quad (9),$$

$$dCz = (C1 + C3) - (C2 + C4) = 0 \quad (20).$$

where, R0 denotes a radius of the circular arc of the bending deformation of the flexible substrate 11; R1 denotes a radius of the bending deformation of the first sensing structure 12 and the second sensing structure 13; R2 denotes a radius of the bending deformation of the third sensing structure 14 and the fourth sensing structure 15; t denotes the thickness of the sensor 1 as shown in FIG. 5A; L1 denotes the length of the circular arc of the bending deformation of the first sensing structure 12 and the second sensing structure 13; and L3 denotes the length of the circular arc of the bending deformation of the third sensing structure 14 and the fourth sensing structure 15.

When the sensor 1 undergoes the bending deformation around the axis parallel to the short axis direction, for example, the first sensing structure 12 and the second sensing structure 13 undergo a bending stretch, and the third sensing structure 14 and the fourth sensing structure 15 undergo a bending compression. At this time, the sum (C1+C2) of the capacitance C1 of the first sensing structure 12 and the capacitance C2 of the second sensing structure 13, and the sum (C3+C4) of the capacitance C3 of the third sensing structure 14 and the capacitance C4 of the fourth sensing structure 15, have an opposite change relationship. At this time, the sum Ctotal of the capacitances of the four sensing structures may counteract changing trends of C1+C2 and C3+C4. That is, Ctotal becomes a constant as shown in equation (8), and Ctotal may not be configured to reflect the bending deformation of the sensor 1 around the axis parallel to the short axis direction.

In some embodiments, the output parameter dCx of the sensor 1 after the bending deformation around the axis parallel to the short axis direction varies linearly in proportion to a bending angle $\beta$, exhibiting extremely high linearity. And dCz and Ctotal are both constants, independent of the bending angle $\beta$. Thus, the sensor 1 is capable of recognizing the bending deformation around the axis parallel to the short axis direction based on the parameters dCx related to the capacitance.

It should be noted that, in addition to the case of the four sensing structures described above, the sensor 1 may have three or any other number of sensing structures distributed on both side surfaces of the flexible substrate 11 in the thickness direction, all extending in the long axis direction. As long as the deformations in different dimensions may be characterized by the parameters related to the resistance (or the capacitance) of the sensing structures, the deformations in a plurality of dimensions may be recognized. Merely by way of example, there are three sensing structures on the sensor 1, two of which are distributed on the upper side of the flexible substrate 11 and the other on the lower side of the flexible substrate 11, at which point the parameters related to the resistance (or the capacitance) of each of the three sensing structures are also related to the bending deformations around the axis parallel to the X-axis (or the Z-axis). At this point, the bending deformations of the sensor 1 around the axis parallel to the X-axis (or the Z-axis) may also be determined by combining the parameters related to the resistance (or the capacitance) produced by each of the three sensing structures.

FIG. 7 is a schematic diagram illustrating a top view of a sensor after a bending deformation around an axis parallel to a thickness direction according to FIG. 5A.

In some embodiments, when the first sensing structure 12, the second sensing structure 13, the third sensing structure 14, and the fourth sensing structure 15 are all the capacitive sensing structures, the sensor 1 is capable of recognizing, based on the parameter dCz related to the capacitance, the bending deformation of the flexible substrate 11 around the axis parallel to the thickness direction.

As shown in FIG. 5A and FIG. 7, when the sensor 1 undergoes the bending deformation around the axis parallel to the thickness direction (e.g., the Z-axis direction), the sensor 1 after the bending deformation is approximated to be a circular arc for ease of illustration. The first sensing structure 12 and the third sensing structure 14 (not shown in FIG. 7) are bent and elongated to L4, and the second sensing structure 13 and the fourth sensing structure 15 (not shown in FIG. 7) are bent and compressed to L2. Considering that the thickness of each of the four sensing structures varies very little, it is approximated that the thickness of each of the four sensing structures is constant for ease of illustration. Then, according to equation (1), the capacitance C1 of the first sensing structure 12 and the capacitance C3 of the third sensing structure 14 change as equation (11) below, and the capacitance C2 of the second sensing structure 13 and the capacitance C4 of the fourth sensing structure 15 change as equation (12) below.

$$C1 = C3 = \varepsilon_0\varepsilon\frac{(L0+L4)w}{2d} = \varepsilon_0\varepsilon\frac{(r0\alpha+r1\alpha)w}{2d} = \varepsilon_0\varepsilon\frac{(2r0+w)\alpha w}{2d} \ (11),$$

$$C2 = C4 = \varepsilon_0\varepsilon\frac{(L0+L2)w}{2d} = \varepsilon_0\varepsilon\frac{(r0\alpha+r2\alpha)w}{2d} = \varepsilon_0\varepsilon\frac{(2r0-w)\alpha w}{2d} \ (12).$$

At this time, Ctotal, dCx, and dCz are determined by the following equations (13), (14), and (15), respectively.

$$Ctotal = C1 + C2 + C3 + C4 = 4\varepsilon_0\varepsilon\frac{L0w}{d} \ (13),$$

$$dCx = (C1 + C2) - (C3 + C4) = 0 \ (14),$$

$$dCz = (C1 + C3) - (C2 + C4) = \frac{2\varepsilon_0\varepsilon w^2}{d}\alpha \ (15),$$

where, r0 denotes a radius of the circular arc of the bending deformation of the flexible substrate 11; r1 denotes a radius of the bending deformation of the first sensing structure 12 and the third sensing structure 14; r2 denotes a radius of the circular arc of the bending deformation of the second sensing structure 13 and the fourth sensing structure 15; L2 denotes a length of the circular arc of the bending deformation of the first sensing structure 12 and the third sensing structure 14; and L4 denotes a length of the circular arc of the bending deformation of the second sensing structure 13 and the fourth sensing structure 15.

In some embodiments, the output parameter dCz of the sensor 1 after the bending deformation around the axis parallel to the thickness direction (e.g., the Z-axis direction) varies linearly with a bending angle $\alpha$, exhibiting extremely high linearity. And dCx and Ctotal are constants, independent of the bending angle $\alpha$. Thus, the sensor 1 is capable of recognizing the bending deformation around the axis parallel to the thickness direction based on the parameter dCz related to the capacitance.

In some embodiments, when the first sensing structure 12, the second sensing structure 13, the third sensing structure 14, and the fourth sensing structure 15 are resistive sensing structures, the processing circuit 16 may identify, based on the parameters related to the resistance of the four sensing structures, the deformation in the at least two dimensions of the flexible substrate 11. Exemplarily, the resistances of the four sensing structures are

$$R1 = \quad R2 = R3 = R4 = \rho\frac{d}{A}$$ . Substituting the areas of the each sensing structures after the deformation of the sensor 1 into the foregoing equation and performing a differential operation yields a similar result for the parameters related to the capacitance, enabling the processing circuit 16 to determine the bending deformation of the sensor 1 around the axis parallel to the thickness direction, the bending deformation around the axis parallel to the short axis direction, and the tensile or compressive deformation in the long axis direction of the flexible substrate. $\rho$ denotes a resistivity of each of the four sensing structures (e.g., the intermediate layer, see below).

In some embodiments, the processing circuit 16 is capable of recognizing a composite deformation where any combination of the following deformations occurs simultaneously: the bending deformation of the sensor 1 around the axis parallel to the thickness direction, the bending deformation around the axis parallel to the short axis direction, and the tensile or compressive deformation in the long axis direction of the flexible substrate 11. For example, the processing circuit 16 is capable of simultaneously determining changes in the parameters related to the capacitance Ctotal, dCx, and dCz, thereby reconstructing components of the deformations in different dimensions simultaneously to restore an actual bending deformation of the sensor 1.

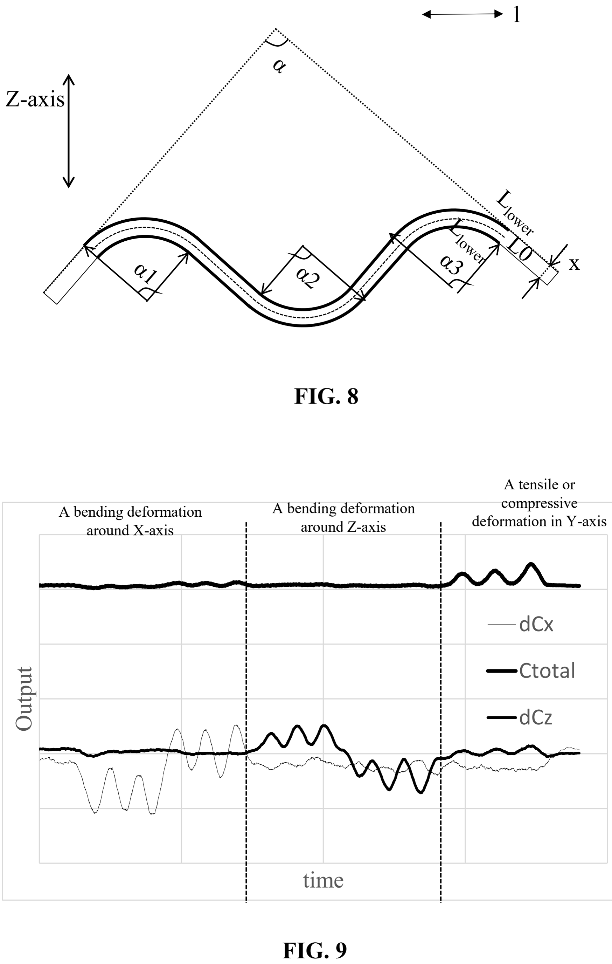

FIG. 8 is a schematic diagram illustrating a flexible sensor with a wrinkle according to some embodiments of the present disclosure. As shown in FIG. 8, when the sensor 1 has a composite deformation in a plurality of dimensions simultaneously, wrinkles may appear on the sensor 1 (FIG. 8 illustrates the wrinkles occurring in a length direction l), which is equivalent to a presence of a plurality of bends (bending angles may include α1, a2, and α3). After geometric integration, it may be seen that α1, α2, and α3 need not be considered anymore, only an angle between the head and tail of the sensor 1 is considered. That is to say, no matter how many times the bending passes in the middle, changes in a length of upper and lower surfaces of the sensor 1 in a thickness direction, $L_{upper} = L0 + \frac{\alpha x}{2}$ with $L_{lower} = L0 - \frac{\alpha x}{2}$ both still hold, while a length L0 of a neutral line remains constant. α is the angle between the head and tail of the sensor 1, and x denotes a width (thickness) of the sensor 1 in a bending direction, indicating that foursensing structures of the sensor 1 effectively prevent the effect of the wrinkles.

FIG. 9 is a schematic diagram illustrating a curve obtained from actual measurement of a sensor according to FIG. 4A.

As shown in FIG. 9, when the sensor 1 undergoes a bending deformation around an axis parallel to a short axis direction (e.g., an X-axis direction), dCx changes significantly with a bending angle, while Ctotal and dCz remain basically stable. When the sensor 1 undergoes a bending deformation around an axis parallel to a thickness direction (e.g., a Z-axis direction), dCz changes significantly with the bending angle, while Ctotal and dCx remain basically stable. When the sensor 1 undergoes a tensile or compressive deformation in a long axis direction (e.g., a Y-axis direction) of the flexible substrate 11, Ctotal changes significantly with a tensile length or a compression length, while dCx and dCz remain basically stable. Thus, when the sensor 1 undergoes the bending deformation, the output parameters related to a capacitance Ctotal, dCx, and dCz in the sensor 1 maintain a high degree of independence and stability, which enables the processing circuit 16 to recognize a deformation in different dimensions based on Ctotal, dCx, and dCz.

In some embodiments, when the sensor 1 is in free bending movement (e.g., there is a compound deformation of the sensor 1), dCx, dCz, and Ctotal all change. At this time, it is possible to decouple the free bending movement of the sensor 1 into the bending deformation around the axis parallel to the thickness direction, the bending deformation around the axis parallel to the short axis direction, and the tensile or compressive deformation in the long axis direction, thereby restoring the actual bending movement of the sensor 1.

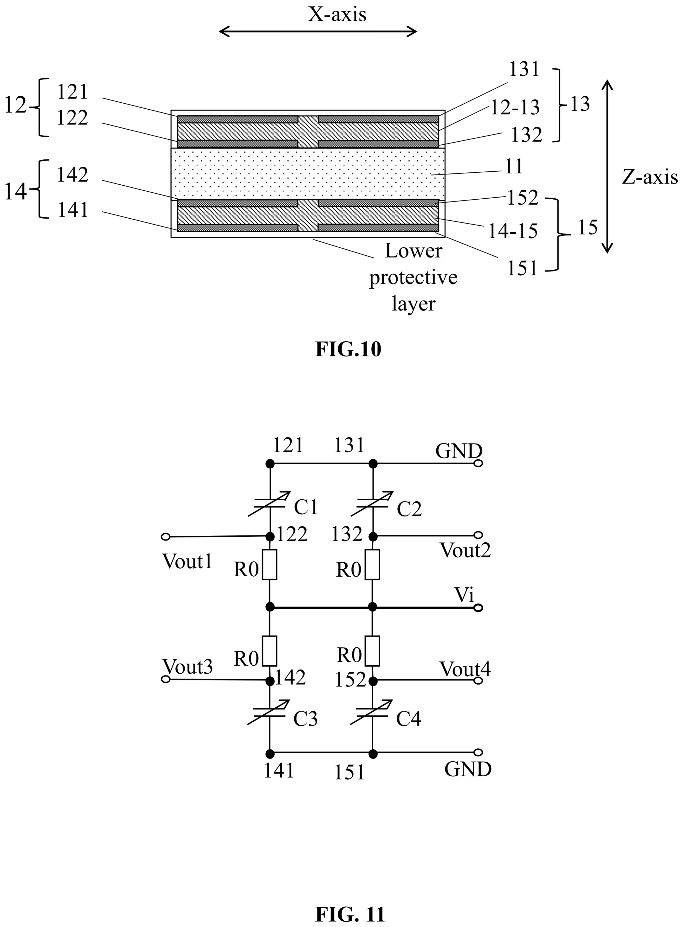

FIG. 10 is a schematic diagram illustrating a cross-section of a sensor according to some embodiments of the present disclosure.

In some embodiments, each of the first sensing structure 12, the second sensing structure 13, the third sensing structure 14, or the fourth sensing structure 15 includes a second conductive layer, an intermediate layer, and a first conductive layer stacked sequentially away from the flexible substrate 11 in a thickness direction. As shown in FIG. 10, the first sensing structure 12 includes a second conductive layer 122, an intermediate layer 12-13, and a first conductive layer 121 stacked sequentially away from the flexible substrate 11. The second sensing structure 13 includes a second conductive layer 132, an intermediate layer 12-13, and a first conductive layer 131 stacked sequentially away from the flexible substrate 11. The third sensing structure 14 includes a second conductive layer 142, an intermediate layer 14-15, and a first conductive layer 141 stacked sequentially away from the flexible substrate 11. The fourth sensing structure 15 includes a second conductive layer 152, an intermediate layer 14-15, and a first conductive layer 151 stacked sequentially away from the flexible substrate 11. In some embodiments, the intermediate layer 12-13 is located between the first conductive layer 121 and the second conductive layer 122 of the first sensing structure 12 (or the first conductive layer 131 and the second conductive layer 132 of the second sensing structure 13). The intermediate layer 14-15 is located between the first conductive layer 141 and the second conductive layer 142 of the third sensing structure 14 (or the first conductive layer 151 and the second conductive layer 152 of the fourth sensing structure 15).

In some embodiments, the first conductive layer and the second conductive layer of each of the sensing structures include an elastic conductive material. The elastic conductive material may enable the first conductive layer and the second conductive layer to be electrically conductive and to revert back to initial shapes when an applied external force disappears. In some embodiments, the elastic conductive material includes, but is not limited to, a conductive adhesive film, a conductive ink, a conductive polymer material, a conductive gel, a liquid metal, or the like. In some embodiments, the first conductive layer and the second conductive layer include the conductive adhesive film, and the conductive adhesive film is made by mixing conductive particles with a polymeric material. In some embodiments, the polymer material includes, but is not limited to, silicone, rubber, resin, or the like. In some embodiments, the first conductive layer and the second conductive layer include the conductive ink, and the conductive ink is made by mixing the conductive particles with an ink material. The conductive ink is capable of being prepared by printing to obtain a pattern with conductivity. In some embodiments, the first conductive layer and the second conductive layer include the conductive polymer material, the conductive gel, the liquid metal, or the like. In some embodiments, the conductive polymeric material includes, but is not limited to, polypyrrole, poly(3,4-ethylenedioxythiophene)-polystyrene sulfonate (PEDOT: PSS), or the like.

In some embodiments, the elastic conductive material includes an elastic material internally filled with conductive particles. By adjusting a density of the conductive particles filled within the elastic material, a conductivity of the first conductive layer and the second conductive layer may be adjusted. In some embodiments, the elastic material includes silicone, rubber, resin, polydimethylsiloxane (PDMS), polyurethane, styrene-butadiene-styrene (SBS), or the like. In some embodiments, the conductive particles include metal powders and carbon nanotubes (e.g., carbon powder, etc.), silver nanowires, carbon black, graphite powder, graphene, or the like.

In some embodiments, the intermediate layers 12-13 and the intermediate layers 14-15 include an elastic insulating material, at which point each sensing structure is a capacitive sensing structure. For example, the elastic insulating material includes silicone, rubber, PDMS, thermoplastic polyurethane (TPU), or the like. In some embodiments, the intermediate layer 12-13 and the intermediate layer 14-15 include a high-resistance elastic material, and a resistivity of the intermediate layer 12-13 and the intermediate layer 14-15 is more than 1000 times greater than a resistivity of the first conductive layer and the second conductive layer, at which point each sensing structure is a composite capacitive-resistive sensing structure.

In some embodiments, thicknesses of the second conductive layer and the first conductive layer of each of the sensing structures range from 1 to 100 $\mu$m.

In some embodiments, the first conductive layer 121 of the first sensing structure 12 and the first conductive layer 131 of the second sensing structure 13 have consistent dimensions and shapes, with widths ranging from 0.5 to 10 mm. In some embodiments, the second conductive layer 122 of the first sensing structure 12 and the second conductive layer 132 of the second sensing structure 13 have consistent dimensions and shapes, with widths ranging from 0.5 to 10 mm. In some embodiments, the first conductive layer 141 of the third sensing structure 14 and the first conductive layer 151 of the fourth sensing structure 15 have consistent dimensions and shapes, with widths ranging from 0.5 to 10 mm. In some embodiments, the second conductive layer 142 of the third sensing structure 14 and the second conductive layer 152 of the fourth sensing structure 15 have consistent dimensions and shapes, with widths ranging from 0.5 to 10 mm.

In some embodiments, the spacing between the first conductive layer 121 of the first sensing structure 12 and the first conductive layer 131 of the second sensing structure 13 ranges from 0.02 to 2 mm. In some embodiments, the spacing between the second conductive layer 122 of the first sensing structure 12 and the second conductive layer 132 of the second sensing structure 13 ranges from 0.02 to 2 mm. In some embodiments, the spacing between the first conductive layer 141 of the third sensing structure 14 and the first conductive layer 151 of the fourth sensing structure 15 ranges from 0.02 to 2 mm. In some embodiments, the spacing between the second conductive layer 142 of the third sensing structure 14 and the second conductive layer 152 of the fourth sensing structure 15 ranges from 0.02 to 2 mm.

In some embodiments, a conductivity of the first conductive layer is greater than a conductivity of the intermediate layer.

The conductivity refers to a parameter used to characterize the ease with which charge flows in a substance. Since the conductivity of the first conductive layer is greater than the conductivity of the second conductive layer, the first conductive layer has better electrical performance. As a result, a resistance of the first conductive layer is less than a resistance of the second conductive layer. It should be noted that, in the present disclosure, the resistance of a given component refers to a resistance between two surfaces of the component spaced apart in the thickness direction of the flexible substrate 11. The resistance of the first conductive layer refers to a resistance between two surfaces of the first conductive layer spaced apart in the thickness direction of the flexible substrate 11, and the resistance of the second conductive layer refers to a resistance between two surfaces of the second conductive layer spaced apart in the thickness direction of the flexible substrate 11. In some embodiments, the conductivity of the first conductive layer may be more than 100 times the conductivity of the second conductive layer. In some embodiments, by setting a density of the conductive particles filled within the elastic material of the first conductive layer greater than a density of the conductive particles filled within the elastic material of the second conductive layer, the conductivity of the first conductive layer may be made greater than the conductivity of the second conductive layer.

When the sensing structure (e.g., the first sensing structure 12, the second sensing structure 13, the third sensing structure 14, and the fourth sensing structure 15) is the composite capacitive-resistive sensing structure or is the capacitive sensing structure, the resistance between two surfaces spaced apart by the intermediate layer in the thickness direction of the flexible substrate 11 is not desirably too small to facilitate the subsequent measurements of the resistance and analysis of the change in value of the resistance. In some embodiments, a resistance between two surfaces of the intermediate layer spaced apart in the thickness direction of the flexible substrate 11 is greater than 0.8 M$\Omega$. For example, when the sensing structure is the composite capacitive-resistive sensing structure, the resistance between the two surfaces of the intermediate layer spaced apart in the thickness direction of the flexible substrate 11 may also be 0.8 M$\Omega$ to 15 G$\Omega$. As another example, when the sensing structure is the capacitive sensing structure, the resistance between the two surfaces of the intermediate layer spaced apart in the thickness direction of the flexible substrate 11 may be great (e.g., greater than 15 G$\Omega$).

By setting the resistance between the two surfaces of the intermediate layer spaced apart in the thickness direction of the flexible substrate 11 to be greater than 0.8 M$\Omega$, the intermediate layer can conduct electricity while maintaining a sufficiently high resistance, which ensures that the measurement of the resistance of the intermediate layer and the analysis of resistance changes can accurately reflect the bending of the sensor 1.

In some embodiments, a relative dielectric constant of the intermediate layer is greater than 2. The relative dielectric constant refers to a physical parameter that characterizes dielectric or polarization properties of a dielectric material. A value of the relative dielectric constant is equal to a ratio of the capacitance of a capacitor of the same dimension made with the corresponding material as the dielectric to that made with a vacuum. The value of the relative dielectric constant is also a characterization of an ability of the material to store electricity, also known as a relative capacitance. Substances with a relative dielectric constant greater than 2 are polar, which means that the intermediate layer with a relative dielectric constant greater than 2 has a certain electrical storage capacity. Thus, the intermediate layer may be equated to a parallel combination of a resistive element and a capacitive element.

In some embodiments, the relative dielectric constant of the intermediate layer may also be greater than or equal to 4. Preferably, the relative dielectric constant of the intermediate layer is greater than 5. In some embodiments, the relative dielectric constant of the intermediate layer is greater than 10.

Setting the relative dielectric constant of the intermediate layer to be greater than 2 allows the intermediate layer to be both the resistive element and the capacitive element. Both the capacitance and the resistance of the intermediate layer change with the deformation of the flexible substrate 11 during the bending of the flexible sensor 1. Thus, parameters of the intermediate layer related to the capacitance and the resistance may reflect the bending of the sensor 1. Reflecting the bending of the sensor 1 by the simultaneous presence of the capacitance and the resistance in this embodiment can result in a significant increase in the sensitivity and accuracy of the sensor 1.

Additionally, by setting the resistance between the two surfaces of the intermediate layer spaced apart in the thickness direction of the flexible substrate 11 to be greater than 0.8 MΩ, it can also be ensured that the intermediate layer not only exhibits excellent resistive properties but also demonstrates superior capacitive performance, thereby ensuring the sensitivity and accuracy of the sensor 1.

In some embodiments, the first conductive layer of each of the first sensing structure, the second sensing structure, the third sensing structure, or the fourth sensing structure is a grounding electrode.

By grounding the first conductive layer of each of the sensing structures, it can act as an electrical shielding layer, which can shield the human body and other sources of interference from the sensor 1 and improve the stability of the sensor 1.

FIG. 11 is an equivalent circuit diagram of a sensor according to FIG. 10.

In some embodiments, the first conductive layer 121 of the first sensing structure 12, the first conductive layer 131 of the second sensing structure 13, the first conductive layer 141 of the third sensing structure 14, and the first conductive layer 151 of the fourth sensing structure 15 are all grounded. The second conductive layer 122 of the first sensing structure 12, the second conductive layer 132 of the second sensing structure 13, the second conductive layer 142 of the third sensing structure 14, and the second conductive layer 152 of the fourth sensing structure 15 are each connected to a voltage output device of the processing circuit 16 through a fixed resistor with a resistance value of R0, to supply the intermediate layer 12-13 and the intermediate layer 14-15 with a pulsed alternating voltage Vi having a peak voltage of Vcc. The first conductive layer 121 of the first sensing structure 12, the first conductive layer 131 of the second sensing structure 13, the first conductive layer 141 of the third sensing structure 14, and the first conductive layer 151 of the fourth sensing structure 15 are each connected to the processing circuit 16 through leads drawn from the flexible substrate 11, to read parameters related to the capacitance of the intermediate layer 12-13 and the intermediate layer 14-15 of each sensing structure. Merely by way of example, the second conductive layer 122 of the first sensing structure 12, the second conductive layer 132 of the second sensing structure 13, the second conductive layer 142 of the third sensing structure 14, and the second conductive layer 152 of the fourth sensing structure 15 may be connected to a voltage detection device to detect voltages Vout1, Vout2, Vout3, and Vout4 output by the first sensing structure 12, the second sensing structure 13, the third sensing structure 14, and the fourth sensing structure 15, respectively. Then an equivalent circuit is shown in FIG. 11, at this time an input Vi signal is a square wave pulse electrical signal with the peak voltage Vcc.

$$\text{Vout(x)} = \text{Vcc} * \left(1 - e^{-\frac{t}{R_0 C(x)}}\right) \ (16).$$

.

Where, Vout(x) denotes an output voltage of the $x_{th}$ sensing structure; x is 1, 2, 3, and 4; Vcc denotes a peak value of the square wave pulse electrical signal Vi as Vcc; R0 denotes the resistance value of the fixed resistor; C(x) denotes a capacitance value of the $x_{th}$ sensing structure (the intermediate layer); and t denotes a measurement time. The magnitude of the output voltage of the $x_{th}$ sensing structure is related to the capacitance of the $x_{th}$ sensing structure (the intermediate layer). In some embodiments, the capacitance value C(x) of each sensing structure may be determined according to equation (16). Based on the capacitance value of the each sensing structure, the parameters (including Ctotal, dCz, and dCx) of the sensor 1 related to the capacitance may be determined. When the sensor 1 undergoes a bending deformation, the processing circuit 16 is capable of recognizing, based on Ctotal, dCx, and dCz, deformations in different dimensions.

In some embodiments, when the intermediate layer of the sensing structure is a high-resistance material, it is equivalent to introducing a parallel resistor R(x) at two ends of the capacitance C(x) of each sensing structure. Thus, the aforementioned equation (16) is modified as follows:

$$\text{Vout(x)} = \frac{R(x)}{R0+R(x)}\text{Vcc} * \left(1 - e^{-\frac{t}{R0C(x)}}\right) \ (17).$$

Where, R(x) denotes a resistance value of the intermediate layer of the $x_{th}$ sensing structure. At this time, the $x_{th}$ sensing structure is a composite capacitive-resistive sensing structure, and the output voltage Vout(x) of the $x_{th}$ sensing structure is a result of a combined effect of the capacitance and the resistance. The processing circuit 16 may still determine the capacitance value C(x) of each sensing structure according to equation (17), thereby determining the parameters related to the capacitance of the sensor 1, including Ctotal, dCz, and dCx. Based on Ctotal, dCx, and dCz, the deformations in different dimensions may be recognized.

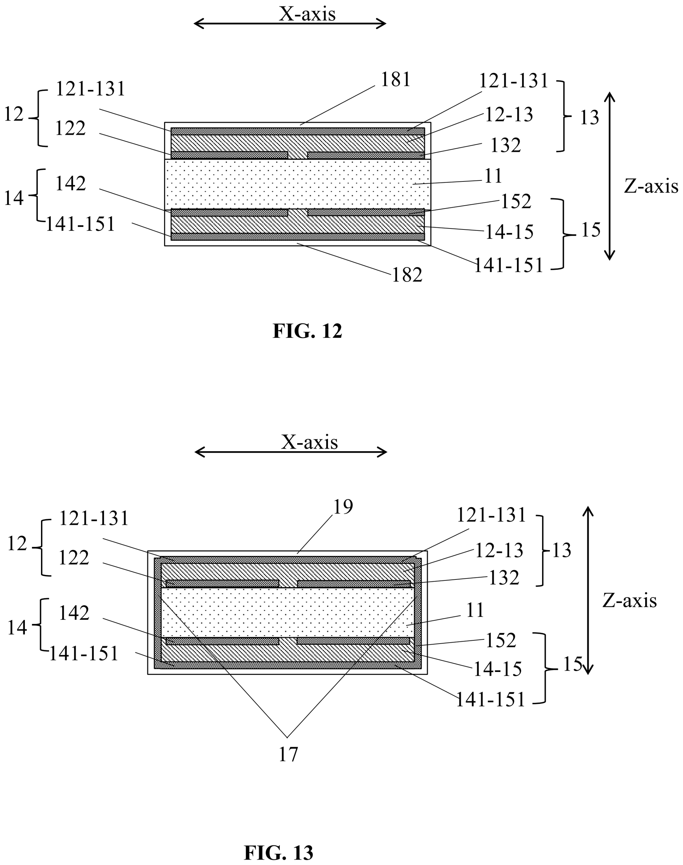

FIG. 12 is a schematic diagram illustrating a cross-section of a sensor according to yet other embodiments of the present disclosure.

In some embodiments, two sensing structures on a same side surface of the flexible substrate 11 share a first conductive layer, and a second conductive layer of the two sensing structures on the same side surface are spaced apart in a short axis direction of the flexible substrate 11. As shown in FIG. 12, the first sensing structure 12 and the second sensing structure 13 share a first conductive layer 121-131. The third sensing structure 14 and the fourth sensing structure 15 share a first conductive layer 141-151. In some embodiments, the first conductive layer 121-13 and the first conductive layer 141-151 have same dimension and same shape, with widths ranging from 0.52 to 12 mm.

In some embodiments, by setting the first conductive layer 121-131 shared by the first sensing structure 12 and the second sensing structure 12 and the first conductive layer 141-151 shared by the third sensing structure 14 and the fourth sensing structure 15 as grounding electrodes, a complete shielding layer can be formed on the upper and lower surfaces of the sensor 1, which achieves an excellent shielding effect against external noise (for example, shielding the sensor 1 from interference sources such as the human body). In addition, the arrangement can effectively simplify the production process of the sensor 1.

FIG. 13 is a schematic diagram illustrating a cross-section of a sensor provided with a connecting member according to FIG. 12.

As shown in FIG. 13, in some embodiments, the sensor 1 further includes a connecting member 17 having electrical conductivity, and the connecting member 17 is provided around a periphery of the flexible substrate 11, connecting with a first conductive layer of each of the sensing structures to form a shielding layer of the sensor 1.

In some embodiments, by using the connecting member 17 to connect the first conductive layer 121-131 and the first conductive layer 141-151 and grounding the first conductive layer 121-131 and the first conductive layer 141-151, a peripheral surface of the flexible substrate 11 is also enclosed, which enables the connecting member 17, the first conductive layer 121-131, and the first conductive layer 141-151 to form a grounding shielding layer that completely encapsulates the flexible substrate 11, thereby achieving the optimal shielding effect against external noise.

In some embodiments, a projection of the first conductive layer of the two sensing structures on the same side surface of the flexible substrate 11 overlies a projection of the second conductive layer of the two sensing structures on the same side surface of the flexible substrate. As shown in FIG. 12 and FIG. 13, a projection of the first conductive layer 121-131 shared by the first sensing structure 12 and the second sensing structure 13 on the flexible substrate 11 overlies a projection of the second conductive layer 122 of the first sensing structure 12 and a projection of the second conductive layer 132 of the second sensing structure 13 on the flexible substrate 11. A projection of the first conductive layer 141-151 shared by the third sensing structure 14 and the fourth sensing structure 15 overlies a projection of the second conductive layer 142 of the third sensing structure 14 and a projection of the second conductive layer 152 of the fourth sensing structure on the flexible substrate 11.

In some embodiments, the projection of the first conductive layer shared by the two sensing structures on the flexible substrate 11 completely overlies the projection of the second conductive layer on the flexible substrate 11, which may effectively reduce a count of pins arranged in the sensor 1, and reduce the difficulty of connecting the processing circuit to the pins.

As shown in FIG. 12, in some embodiments, the first conductive layer of the each of the first sensing structure 12, the second sensing structure 13, the third sensing structure 14, or the fourth sensing structure 15 is covered with a first protective structure on a side surface away from the flexible substrate 11. In some embodiments, the first protective structure includes an upper protective layer 181 and a lower protective layer 182.

As shown in FIG. 13, in some embodiments, an exposed surface of the flexible substrate 11 is covered with a second protective structure 19.

In some embodiments, the upper protective layer 181, the lower protective layer 182, and the second protective structure 19 are insulating and resilient flexible materials or high resistance and resilient flexible materials. In some embodiments, the upper protective layer 181, the lower protective layer 182, and the second protective structure 19 all include, but are not limited to, silicone, PDMS, TPU, or the like. In some embodiments, the upper protective layer 181, the lower protective layer 182, and the second protective structure 19 have a resistivity greater than or equal to a resistivity of the flexible substrate 11. In some embodiments, the upper protective layer 181 and the lower protective layer 182 both have thicknesses ranging from 1 to 100 um.

FIG. 12 and FIG. 13 illustrate the difference between the first protective structure and the second protective structure 19, respectively. In the embodiment illustrated in FIG. 12, the first protective structure is formed by the upper protective layer 181 and the lower protective layer 182, which respectively overlie and protect all edges of all conductive layers of the first sensing structure 12 and the second sensing structure 13, and all edges of all conductive layers of the third sensing structure 14 and the fourth sensing structure 15. And in the embodiment shown in FIG. 13, the second protective structure 19 simultaneously overlies and protects all edges of all conductive layers of the four sensing structures.

As shown in FIG. 12, in some embodiments, the upper protective layer 181 encompasses all edges of the first conductive layer 121-131 shared by the first sensing structure 12 and the second sensing structure 13, the second conductive layer 122 of the first sensing structure 12, and the second conductive layer 132 of the second sensing structure 13. In some embodiments, the lower protective layer 182 encompasses all edges of the first conductive layer 141-151 shared by the third sensing structure 14 and the fourth sensing structure 15, the second conductive layer 142 of the third sensing structure 14, and the second conductive layer 152 of the fourth sensing structure 15. By incorporating the first protective structure, it is possible to effectively prevent damage to the internal sensing structures of the sensor 1 during use, and to avoid introducing additional noise caused by contact with external conductors (such as the human body, liquids, etc.).

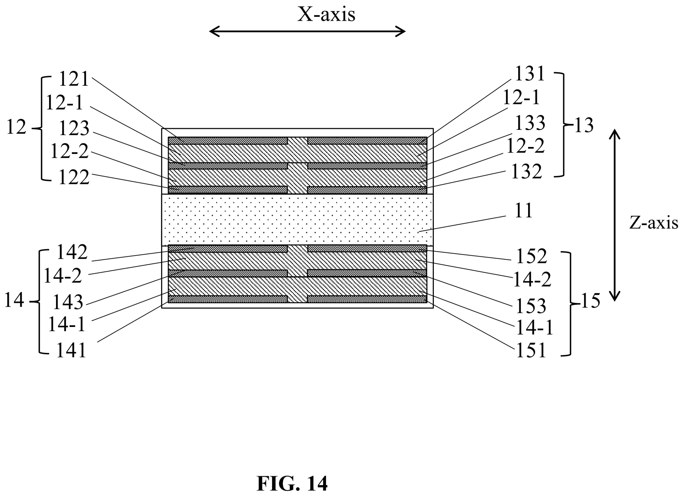

FIG. 14 is a schematic diagram illustrating a cross-section of a sensor according to some other embodiments of the present disclosure.

In some embodiments, each of the first sensing structure 12, the second sensing structure 13, the third sensing structure 14, or the fourth sensing structure 15 includes a second conductive layer, a second intermediate layer, a third conductive layer, a first intermediate layer, and a first conductive layer stacked sequentially away from the flexible substrate 11 in a thickness direction. As shown in FIG. 14, the first sensing structure 12 includes the second conductive layer 122, a second intermediate layer 12-2, a third conductive layer 123, a first conductive layer 12-1, and the first conductive layer 12-1 stacked sequentially away from the flexible substrate 11 in the thickness direction. The second sensing structure 13 includes the second conductive layer 132, a second intermediate layer 13-2, a third conductive layer 133, a first intermediate layer 13-1, and the first conductive layer 131 stacked sequentially away from the flexible substrate 11 in the thickness direction. The third sensing structure 14 includes the second conductive layer 142, a second intermediate layer 14-2, a third conductive layer 143, a first intermediate layer 14-1, and the first conductive layer 141 stacked sequentially away from the flexible substrate 11 in the thickness direction. The fourth sensing structure 15 includes the second conductive layer 152, a second intermediate layer 15-2, a third conductive layer 153, a first intermediate layer 15-1, and the first conductive layer 151 stacked sequentially away from the flexible substrate 11 in the thickness direction.

In some embodiments, parameters of the third conductive layer, the first conductive layer, and the second conductive layer of each of the sensing structures are the same. Parameters of the second intermediate layer, the first intermediate layer, and an intermediate layer described above of each of the sensing structures are the same. For specific materials and dimensional parameters of each structure, refer to FIG. 10 and related descriptions.

In some embodiments, the sensitivity of the sensor 1 in detecting bending deformations in various dimensions can be effectively improved by providing more conductive layers (e.g., the third conductive layer of FIG. 14) in a stacked manner in the thickness direction of each sensing structure.

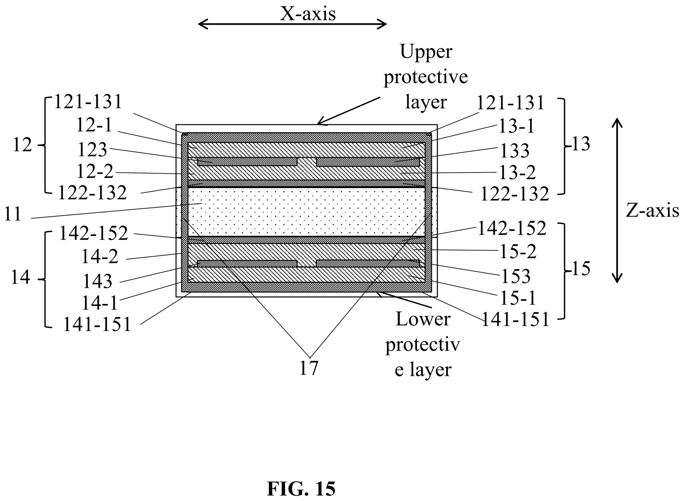

FIG. 15 is a schematic diagram illustrating a cross-section of a sensor according to some further embodiments of the present disclosure.

As shown in FIG. 15, in some embodiments, two sensing structures on a same side surface of the flexible substrate 11 share a first conductive layer or a second conductive layer, and third conductive layers of the two sensing structures on the same side surface are spaced apart in a short axis direction of the flexible substrate 11. In some embodiments, the first conductive layer and the second conductive layer of each of the first sensing structure 12, the second sensing structure 13, the third sensing structure 14, or the fourth sensing structure 15 are both grounding electrodes.

In some embodiments, the first conductive layer 121-131 and the second conductive layer 122-132 shared by the first sensing structure 12 and the second sensing structure 13, and the first conductive layer 141-151 and the second conductive layer 142-152 shared by the third sensing structure 14 and the fourth sensing structure 15, are all grounded. The first conductive layer 122-132 and the second conductive layer 142-152 are respectively connected to a voltage output device of the processing circuit 16 through a fixed resistor, to provide a pulsed alternating voltage to the first intermediate layer 12-1, the second intermediate layer 12-2, the first intermediate layer 14-1, and the second intermediate layer 14-2. The first conductive layer 121-131 and the second conductive layer 122-132 shared by the first sensing structure 12 and the second sensing structure 13, and the first conductive layer 141-151 and the second conductive layer 142-152 shared by the third sensing structure 14 and the fourth sensing structure 15, are each connected to the processing circuit 16 through leads drawn from the flexible substrate 11 to read parameters (e.g., Ctotal, dCz, and dCx) related to a capacitance of each of the sensing structures (e.g., each of the first intermediate layer and the second intermediate layer), enabling the processing circuit 16 to be configured to recognize deformations of the sensor 1 in different dimensions. In the embodiments shown in FIG. 14 and FIG. 15, the sensor 1 also has a first protective structure or the second protective structure 19, as shown in FIG. 12 and FIG. 13, overlying and protecting all edges of all conductive layers of each sensing structure.

As shown in FIG. 15, in some embodiments, the sensor 1 also includes the connecting member 17. The connecting member 17 is provided around a periphery of the sensor 1, and two ends of the connecting member 17 are connected to the first conductive layers on both side surfaces of the flexible substrate 11 in the thickness direction, respectively.

In some embodiments, the first conductive layer 121-131 and the first conductive layer 141-151 on both side surfaces of the flexible substrate 11 in the thickness direction are connected and grounded through the connecting member 17, enclosing a peripheral surface of the flexible substrate 11. The arrangement allows the connecting member 17, the first conductive layer 121-131, and the first conductive layer 141-151 to form a grounded shielding layer that completely encloses the flexible substrate 11, achieving better shielding against external noise.

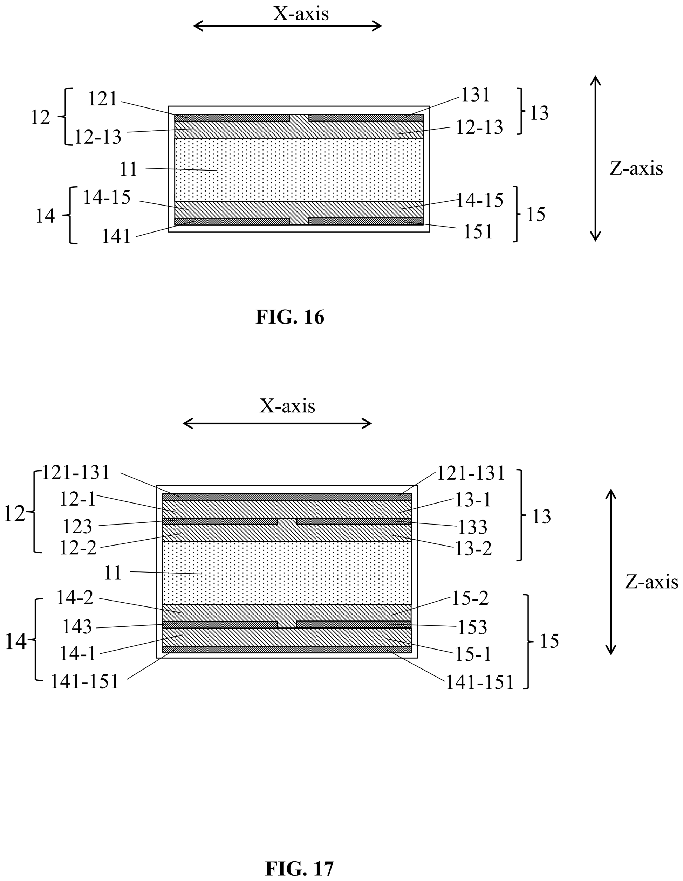

FIG. 16 is a first schematic diagram illustrating a cross-section of a sensor according to some embodiments of the present disclosure.

In some embodiments, each of the first sensing structure 12, the second sensing structure 13, the third sensing structure 14, or the fourth sensing structure 15 includes an intermediate layer and a first conductive layer stacked sequentially away from the flexible substrate 11 in a thickness direction, and the flexible substrate 11 is made of an elastic conductive material.

As shown in FIG. 16, the flexible substrate 11 made of the elastic conductive material may conduct electricity, thereby effectively reducing the use of conductive layers in each sensing structure, which allows the sensor 1 to avoid the need for excessive conductive layers, effectively simplifying the preparation of the sensing structure.

As shown in FIG. 16, in some embodiments, a conductivity of the flexible substrate 11 is greater than a conductivity of the intermediate layer. Since the conductivity of the flexible substrate 11 is greater than the conductivity of the intermediate layer (including the intermediate layer 12-13 and the intermediate layer 14-15), the flexible substrate 11 has better electrical performance. As a result, a resistance of the flexible substrate 11 of each of the sensing structures is less than a resistance of the intermediate layer. The resistance of the flexible substrate 11 refers to a resistance between two surfaces of the flexible substrate 11 spaced apart in the thickness direction, and the resistance of the intermediate layer refers to a resistance between two surfaces of the intermediate layer spaced apart in the thickness direction. In some embodiments, the conductivity of the flexible substrate 11 may be more than 100 times the conductivity of the intermediate layer. In some embodiments, by setting a density of conductive particles filled within an elastic material of the flexible substrate 11 greater than a density of conductive particles filled within an elastic material of the intermediate layer, it is possible to make the conductivity of the flexible substrate 11 greater than the resistivity of the intermediate layer.

In some embodiments, the resistance between two surfaces of the intermediate layer spaced apart in the thickness direction is greater than 0.8 MΩ.

In some embodiments, a relative dielectric constant of the intermediate layer is greater than 2. The intermediate layer may be equivalently represented as a parallel combination of a resistive element and a capacitive element. In some embodiments, the relative dielectric constant of the intermediate layer may also be greater than or equal to 4. Preferably, the relative dielectric constant of the intermediate layer is greater than 5. In some embodiments, the relative dielectric constant of the intermediate layer is greater than 10.

As shown in FIG. 16, in some embodiments, the first conductive layer of the each of the first sensing structure 12, the second sensing structure 13, the third sensing structure 14, or the fourth sensing structure 15 is a grounding electrode. In some embodiments, the first conductive layers of two sensing structures on a same side surface of the flexible substrate 11 are spaced apart in a short axis direction of the flexible substrate 11.

In some embodiments, by grounding the first conductive layer of each sensing structure, it can function as an electrical shielding layer, shielding the sensor 1 from interference sources such as the human body, thereby improving the stability of the sensor 1. In some embodiments, by setting the first conductive layer of each sensing structure to be the grounding electrode, a complete shielding layer can be formed on the upper and lower surfaces of the sensor 1, achieving excellent shielding of external noise. Additionally, the arrangement can effectively simplify the production process of the sensor 1.

In some embodiments, the first conductive layer 121 of the first sensing structure 12, the first conductive layer 131 of the second sensing structure 13, the first conductive layer 141 of the third sensing structure 14, and the first conductive layer 151 of the fourth sensing structure 15 are grounded. The flexible substrate 11 is connected to a voltage output device of the processing circuit 16 through a fixed resistor, so as to supply a pulsed alternating voltage to the intermediate layer 12-13 and the intermediate layer 14-15. The first conductive layer 121 of the first sensing structure 12, the first conductive layer 131 of the second sensing structure 13, the first conductive layer 141 of the third sensing structure 14, and the first conductive layer 151 of the fourth sensing structure 15, are each connected to the processing circuit 16 through leads drawn from the flexible substrate 11 to read parameters (e.g., Ctotal, dCz, and dCx) related to a capacitance of each of the sensing structures (e.g., the intermediate layer), enabling the processing circuit 16 to be used to recognize deformations of the sensor 1 in different dimensions. In the embodiment illustrated in FIG. 16, the sensor 1 also has a first protective structure or the second protective structure 19, as illustrated in FIG. 12 and FIG. 13, overlying and protecting all edges of all conductive layers of each sensing structure.

FIG. 17 is a second schematic diagram illustrating a cross-section of a sensor according to some embodiments of the present disclosure.

As shown in FIG. 17, in some embodiments, each of the first sensing structure 12, the second sensing structure 13, the third sensing structure 14, or the fourth sensing structure 15 includes a second intermediate layer, a third conductive layer, a first intermediate layer, and a first conductive layer stacked sequentially away from the flexible substrate 11 in a thickness direction. In some embodiments, two sensing structures on a same side surface of the flexible substrate 11 share the first conductive layer, and the third conductive layers of the two sensing structures on the same side surface are spaced apart in a short axis direction of the flexible substrate 11.

As shown in FIG. 17, in some embodiments, parameters of the third conductive layer of each sensing structure are consistent with parameters of a shared first conductive layer. Parameters of the second intermediate layer and the first intermediate layer of each sensing structure are consistent with parameters of the intermediate layer described above. For specific materials and dimensional parameters of each structure, please refer to FIG. 10 and related descriptions.

In some embodiments, a projection of the first conductive layer on the flexible substrate 11 shared by the two sensing structures completely overlies a projection of the third conductive layer on the flexible substrate 11, which can effectively reduce a count of pins arranged in the sensor 1, and reduce the difficulty in connecting the pins of the processing circuit.

As shown in FIG. 17, in some embodiments, the first conductive layer 121-131 shared by the first sensing structure 12 and the second sensing structure 13, and the first conductive layer 141-151 shared by the third sensing structure 14 and the fourth sensing structure 15 are grounded. The third conductive layer of each of the sensing structures is connected to a voltage output device of the processing circuit 16 through a fixed resistor to supply a pulsed alternating voltage for the first intermediate layer 12-1, the second intermediate layer 12-2, the first intermediate layer 14-1, and the second intermediate layer 14-2.

The first conductive layer 121-131 shared by the first sensing structure 12 and the second sensing structure 13, the first conductive layer 141-151 shared by the third sensing structure 14 and the fourth sensing structure 15, are each connected to the processing circuit 16 through leads drawn from the flexible substrate 11 to read parameters (e.g., Ctotal, dCz, and dCx) related to a capacitance of the each sensing structure (e.g., each of the first intermediate layer and the second intermediate layer), enabling the processing circuit 16 to recognize deformations in different dimensions of the sensor 1.

In some embodiments, both the first conductive layer and the flexible substrate 11 are grounding electrodes.

In some embodiments, by grounding the first conductive layer and the flexible substrate 11 of each sensing structure, it can function as an electrical shielding layer, which can shield the human body and other sources of interference from the sensor 1 and improve the stability of the sensor 1. In the embodiment illustrated in FIG. 17, the sensor 1 also has a first protective structure or the second protective structure 19, as illustrated in FIG. 12 and FIG. 13, overlying and protecting all edges of all conductive layers of each sensing structure, shielding the external environment from noise interference.

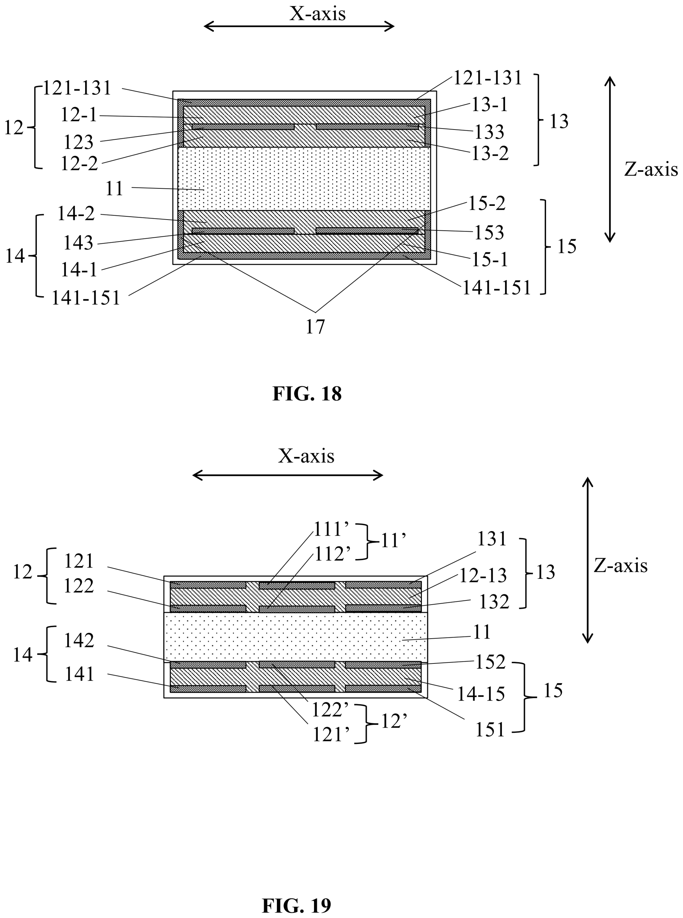

FIG. 18 is a third schematic diagram illustrating a cross-section of a sensor according to some embodiments of the present disclosure.

As shown in FIG. 18, in some embodiments, the sensor 1 further includes the connecting member 17, the connecting member 17 is provided around two sensing structures on a same side surface of the flexible substrate 11, one end of the connecting member 17 is connected to a first conductive layer of the two sensing structures on the same side surface, and the other end of the connecting member 17 is connected to a side surface of the flexible substrate 11 where the two sensing structures are located.

In some embodiments, the flexible substrate 11 and the shared first conductive layer on the same side surface of the two sensing structures are connected and grounded through the connecting member 17, which closes off the periphery of the intermediate layer of each of the sensing structures so that the connecting member 17, the flexible substrate 11, and the shared first conductive layer form a grounded shielding layer that completely wraps the sensing structures, achieving the best shielding effect against external noise.

FIG. 19 is a fourth schematic diagram illustrating a cross-section of a sensor according to some embodiments of the present disclosure.

In some embodiments, the sensor 1 further includes a plurality of sensing structures arranged in a width direction of the flexible substrate 11. As shown in FIG. 19, the sensor 1 includes a fifth sensing structure 11' and a sixth sensing structure 12'. The fifth sensing structure 11' and the sixth sensing structure 12' each include a multilayer structure arranged on two side surfaces in a thickness direction. Each layer of the multilayer structure is stacked in the thickness direction. For example, the fifth sensing structure 11' includes a first conductive layer 111' and a second conductive layer 112'. The sixth sensing structure 12' includes a first conductive layer 121' and a second conductive layer 122'. As shown in FIG. 19, in some embodiments, the multilayer structure includes a second conductive layer, an intermediate layer, and a first conductive layer stacked sequentially away from the flexible substrate 11.

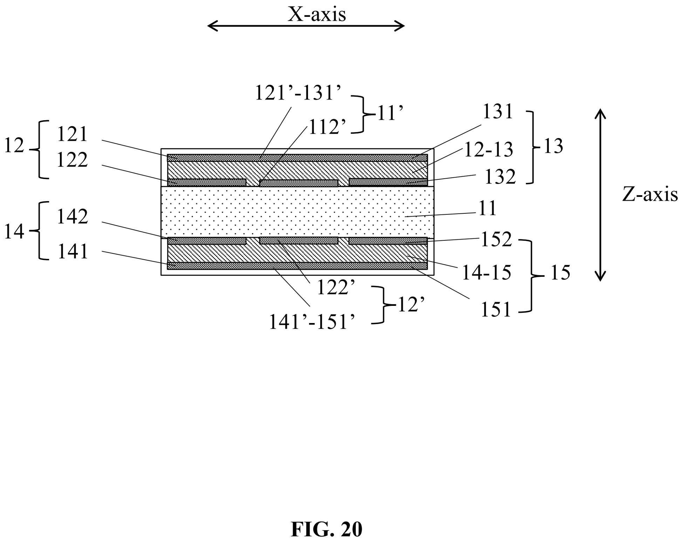

FIG. 20 is a fifth schematic diagram illustrating a cross-section of a sensor according to some embodiments of the present disclosure.

In some embodiments, three sensing structures on a same side surface of the flexible substrate 11 share a first conductive layer, and second conductive layers of the three sensing structures are spaced apart in a short axis direction of the flexible substrate 11. As shown in FIG. 20, in some embodiments, the first sensing structure 12, the second sensing structure 13, and the fifth sensing structure 11' share a first conductive layer 121'-131'. In some embodiments, the third sensing structure 14, the fourth sensing structure 15, and the sixth sensing structure 12' share a first conductive layer 141'-151'. In some embodiments, the first conductive layers 121'-131' and the first conductive layers 141'- 151' have consistent dimensions and shapes, with widths ranging from 0.52 to 12 mm. In the embodiment shown in FIG. 20, parameters of materials, shapes, and dimensions of the remaining structures of the sensor 1 remain unchanged. For specific details, please refer to the descriptions provided above.

In some embodiments, the flexible substrate 11 includes the short axis direction (e.g., an X-axis direction) and a long axis direction (e.g., a Y-axis direction) perpendicular to a thickness direction (e.g., a Z-axis direction), and a ratio of dimensions of the sensor 1 in the short axis direction to dimensions in the thickness direction ranges from 1:1 to 10:1.

The sensor 1 is difficult to prepare if the ratio of the dimensions of the sensor 1 in the short axis direction to the dimensions of the sensor 1 in the thickness direction is too small.

The ratio of the dimensions of the sensor 1 in the short axis direction to the dimensions in the thickness direction is too large, which results in wrinkles in the sensor 1 when the sensor 1 undergoes a bending deformation around an axis parallel to the short axis direction. The wrinkles may break symmetry of each sensing structure, causing an output (e.g., a output voltage) of the sensing structure to be unstable, making it impossible for the sensing structure on the sensor 1 to do a composite differential to cancel out the interference. In some embodiments, the ratio of the dimensions of the sensor 1 in the short axis direction to the dimensions in the thickness direction is within 55:1. Preferably, the ratio of the dimensions of the sensor 1 in the short axis direction to the dimensions in the thickness direction is 2:1 or 3:1.

In some embodiments, the processing circuit 16 reads first parameters related to a resistance or a capacitance of each sensing structure, respectively, and determines a deformation in at least two dimensions of the flexible substrate 11 based on the parameters. In some embodiments, the first parameters are a current value or a voltage value, and the processing circuit 16 determines the deformation in the at least two dimensions of the flexible substrate 11 based on the current value or the voltage value. In some embodiments, the processing circuit 16 determines a capacitance value or a resistance value of each of the first sensing structure and the second sensing structure based on the current value or the voltage value, and determines the deformation in the at least two dimensions of the flexible substrate 11 based on the capacitance value or the resistance value.

In some embodiments, the processing circuit 16 determines the deformation in at least two dimensions of the flexible substrate 11 directly based on the current value or voltage value of each sensing structure. For example, based on a mapping relationship between the current value or the voltage value and deformation variables in the different dimensions of the flexible substrate 11, the processing circuit 16 obtains the deformation in the different dimensions of the flexible substrate 11 by measuring the current value or the voltage value of the each sensing structure. As another example, a machine learning model is trained with a historical current value or a historical voltage value and deformations on historical dimensions of the flexible substrate 11, and the processing circuit 16 inputs measured parameters (e.g., an actual current value or a historical actual value) of the each sensing structure into the machine learning model to obtain the deformations in the different dimensions of the flexible substrate 11.

In some embodiments, based on the mapping relationship between the capacitance or the resistance and the deformation variables in the different dimensions of the flexible substrate 11, the processing circuit 16 determines the capacitance or the resistance of the sensing structure based on the current value or the voltage value, and then based on the mapping relationship, determines the deformations in the different dimensions of the flexible substrate 11. In some embodiments, the processing circuit 16 determines the capacitance or the resistance of the sensing structure from a historical current value or a historical voltage value, trains the machine learning model based on the capacitance or the resistance and the deformations in the historical dimensions of the flexible substrate 11, determines the capacitance or the resistance based on the measured parameters (e.g., the actual current value or the historical actual value) of the each sensing structure, and inputs the capacitance or the resistance into the machine learning model to obtain the deformations in the different dimensions of the flexible substrate 11.

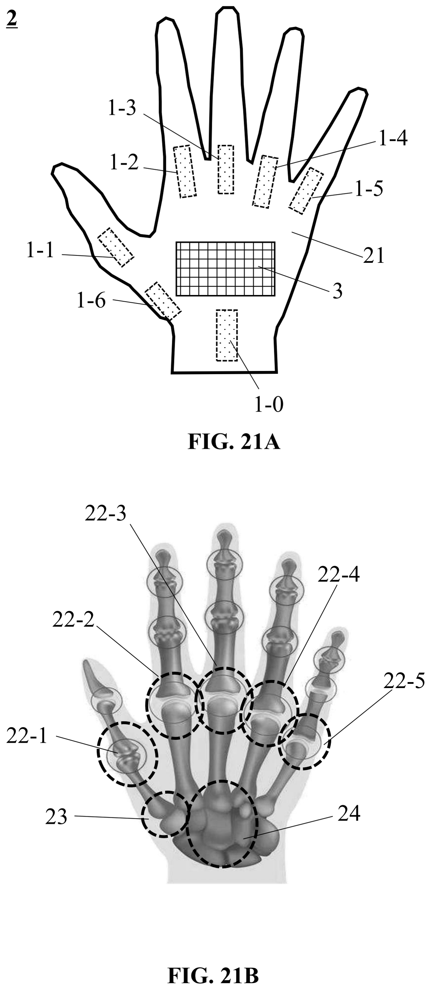

FIG. 21A is a schematic diagram illustrating a structure of a smart glove according to some embodiments of the present disclosure. FIG. 21B is a schematic diagram illustrating joints of a user's hand according to some embodiments of the present disclosure.

As shown in FIG. 21A, in some embodiments, a smart glove 2 includes a glove body 21, the sensor 1, and a processor 3 configured to receive and process data collected by the sensor 1. The sensor 1 being located in any one or more regions of the glove body 21 corresponding to finger joints 22, carpometacarpal joints 23, and wrist joints 24 of a user.

The smart glove 2 refers to a device that is used to capture the motion of hand or fingers of a user. For example, the smart glove 2 includes a motion capture glove, a sign language glove, a virtual reality (VR) glove, a force feedback glove, a haptic feedback glove, etc. As shown in FIG. 21A and FIG. 21B, in some embodiments, the sensor 1-1 is placed at the back of a finger joint (e.g., metacarpophalangeal joints) 22-1 of the thumb in the glove body 21. The sensor 1-2 is placed at the back of a finger joint 22-2 of the index finger in the glove body 21. The sensor 1-3 is placed at the back of a finger joint 22-3 of the middle finger in the glove body 21. The sensor 1- 4 is placed at the back of a finger joint 22-4 of the ring finger in the glove body 21. The sensor 1-5 is placed at the back of a finger joint 22-5 of the pinky finger in the glove body 21. Since the root joint of the hand may both bend and swing laterally, possessing bending motion information in two dimensions, and the back of the finger also undergoes axial stretching due to bending, the characteristics align with the sensitivity of the sensor 1. As a result, the sensor 1 may achieve complete and precise motion capture of the hand or fingers, and smooth and natural motion reproduction.

As shown in FIG. 21A and FIG. 21B, in some embodiments, the sensor 1-6 is placed at the dorsal aspect of the carpometacarpal joint 23 in the glove body 21 at the point where the metacarpal bone of the thumb is connected to the wrist. In some embodiments, the sensor 1-0 may be placed at the back of the wrist joint 24, or at a side surface or front of the wrist joint 24. Similarly, the carpometacarpal joint 23 and the wrist joint 24 of the hand may both bend and swing laterally, possessing bending motion information in two dimensions, which allows the sensor 1-0 and the sensor 1-6 to capture and reproduce the motion of the wrist joint and the carpometacarpal joint of the hand, respectively. Since the other finger joints have only one dimension of bending motion, the sensor 1 in the presently illustrated embodiment and other flexible sensors may be used. In some embodiments, the other flexible sensors include, but are not limited to, inductive, resistive, capacitive, fiber optic, or the like. Complete hand motion capture and restoration of the user may be realized by multi-sensor cooperation.

In some embodiments, data from the sensor 1 is transmitted to a control chip of the processor 3 via a wire, so as to carry out the analysis and processing of the motion data of the smart glove 2 and interact with an upper computer such as a computer, a cell phone, a VR device, or the like. For example, interactions include controlling on/off switching of external devices, volume adjustments, program progression of external devices (e.g., controlling the movement of a game character), fitness feedback, or the like.

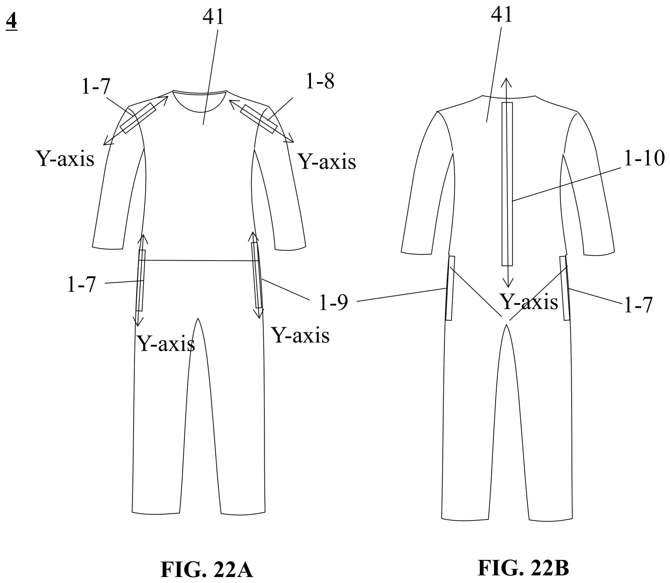

FIG. 22A is a front schematic diagram illustrating a smart garment according to some embodiments of the present disclosure. FIG. 22B is a rear schematic diagram illustrating a smart garment according to some embodiments of the present disclosure.

In some embodiments, a smart garment 4 includes a garment body 41, a sensor 1, and a processor configured to receive and process data collected by the sensor 1. The sensor 1 is located in any one or more regions of the garment body corresponding to shoulder joints, spine joints, hip joints, and ankle joints of a user.

The smart garment 4 refers to a device for capturing body movements of the user. As shown in FIG. 22A and FIG. 22B, in some embodiments, the sensor 1-7 and the sensor 1-8 are placed at the front or the back of the shoulder joints on two sides above the shoulders of the user, respectively, to enable motion capture of shoulders of the user. In some embodiments, a length range of the sensor 1-7 and the sensor 1-8 in a Y-axis direction covers a width of the shoulder joint and spans a brachial circumference such that the brachial circumference on each side of the shoulder is within 1/3 of the length of the sensor 1-7 and the sensor 1-8 in the Y-axis direction, respectively. In some embodiments, the length of the sensor 1-7 and the sensor 1-8 in the Y-axis direction is greater than 10 cm. As shown in FIG. 22B, in some embodiments, the sensor 1-10 is placed at the spine joint directly in the middle of the back of the user, and a length range of the sensor 1-10 in the Y-axis direction covers the entire spine of the user (e.g., from the tailbone to the neck). In some embodiments, the length of the sensor 1-10 in the Y-axis direction is greater than 20 cm. In some embodiments, the sensor 1-7 and the sensor 1-9 are placed at the hip joints on each side of the user body (e.g., on either side of the hip or on both the front and back sides of the hip). In some embodiments, a length range of the sensor 1-7 and the sensor 1-9 cover the entire hip joint in the Y-axis direction. In some embodiments, the length of the sensor 1-7 and the sensor 1-9 in the Y-axis direction is greater than 10cm.

Since other joints of the body (e.g., elbow, knee, ankle, etc.) have bending motion in only one dimension, the sensor 1 in the illustrated embodiment and other flexible sensors may be used. In some embodiments, the other flexible sensors include, but are not limited to, inductive, capacitive, resistive, or the like. Complete hand motion capture and restoration of the user may be realized by multi-sensor cooperation.

In some embodiments, the data from the sensor 1 is transmitted to a control chip of the processor via a wire, so as to carry out the analysis and processing of the movement data of the smart garment 4 and interact with an upper computer such as a computer, a cell phone, a VR device, etc. For example, interactions include controlling on/off switching of external devices, volume adjustments, program progression of external devices (e.g., controlling the movement of a game character), fitness feedback, or the like.

The basic concepts have been described above, and it is apparent to a person skilled in the art that the above detailed disclosure serves only as an example and does not constitute a limitation of the present disclosure. While not expressly stated herein, various modifications, improvements, and amendments may be made to the present disclosure by those skilled in the art. Those types of modifications, improvements, and amendments are suggested in the present disclosure, so those types of modifications, improvements, and amendments remain within the spirit and scope of the exemplary embodiments of the present disclosure.

**Claims**

1. A sensor, comprising:

   a flexible substrate;
   a first sensing structure and a second sensing structure, wherein the first sensing structure and the second sensing structure each include a multilayer structure arranged on a same side surface of the flexible substrate in a thickness direction, each layer of the multilayer structure is stacked in the thickness direction; and
   a processing circuit, wherein the processing circuit reads first parameters, each of which related to a resistance or a capacitance of the first sensing structure and the second sensing structure, respectively, and determines a deformation in at least two dimensions of the flexible substrate based on the first parameters.

2. The sensor of claim 1, wherein the first sensing structure and the second sensing structure are distributed on both sides of the flexible substrate in the thickness direction, and a projection of the first sensing structure on the flexible substrate at least partially overlaps with a projection of the second sensing structure on the flexible substrate.

3. The sensor of claim 2, wherein the flexible substrate includes a short axis direction and a long axis direction both perpendicular to the thickness direction, and the deformation in the at least two dimensions includes a bending deformation around an axis parallel to the short axis direction and a tensile or compressive deformation in the long axis direction.

4. The sensor of claim 1, wherein the flexible substrate includes a long axis direction perpendicular to the thickness direction, the first sensing structure and the second sensing structure are distributed on the same side surface of the flexible substrate in the thickness direction, and the first sensing structure and the second sensing structure are disposed side by side and both extend in the long axis direction of the flexible substrate.

5. The sensor of claim 4, wherein the deformation in the at least two dimensions includes a bending deformation around an axis parallel to the thickness direction and a tensile or compressive deformation in the long axis direction.

6. The sensor of claim 1, further comprising a third sensing structure and a fourth sensing structure, the third sensing structure and the fourth sensing structure each include a multilayer structure arranged on a same side surface in the thickness direction, each layer of the multilayer structure is stacked in the thickness direction;
   wherein the flexible substrate includes a long axis direction and a short axis direction both perpendicular to the thickness direction, the first sensing structure and the second sensing structure are distributed side by side on one side surface of the flexible substrate in the thickness direction and both extend in the long axis direction; and the third sensing structure and the fourth sensing structure are distributed side by side on the other side surface of the flexible substrate in the thickness direction and both extend in the long axis direction.

7. The sensor of claim 6, wherein the processing circuit reads second parameters each of which related to a resistance or a capacitance of the third sensing structure and the fourth sensing structure respectively, and determines the deformation in the at least two dimensions of the flexible substrate based on the first parameters each of which related to the resistance or the capacitance of the first sensing structure and the second sensing structure and the second parameters each of which related to the resistance or the capacitance of the third sensing structure and the fourth sensing structure.

8. The sensor of claim 7, wherein the deformation in the at least two dimensions includes a bending deformation around an axis parallel to the thickness direction, a bending deformation around an axis parallel to the short axis direction, and a tensile or compressive deformation in the long axis direction of the flexible substrate.

9. The sensor of claim 6, wherein the first sensing structure and the second sensing structure are distributed symmetrically about a first mid-section, and the third sensing structure and the fourth sensing structure are distributed symmetrically about the first mid-section, wherein the first mid-section represents a mid-section in the flexible substrate parallel to a plane formed by the thickness direction and the long axis direction; and

the first sensing structure and the third sensing structure are distributed symmetrically about a second mid-section of the flexible substrate, the second sensing structure and the fourth sensing structure are distributed symmetrically about the second mid-section, wherein the second mid-section represents a mid-section in the flexible substrate parallel to a plane formed by a short axis direction and the long axis direction.

10. The sensor of any one of claims 1-6, and 9, wherein each of the first sensing structure, the second sensing structure, the third sensing structure, or the fourth sensing structure includes a second conductive layer, an intermediate layer, and a first conductive layer stacked sequentially away from the flexible substrate in the thickness direction.

11. The sensor of claim 10, wherein a conductivity of the first conductive layer is greater than a conductivity of the intermediate layer.

12. The sensor of claim 10, wherein a resistance between two surfaces of the intermediate layer spaced apart in the thickness direction of the flexible substrate is greater than 0.8 MΩ.

13. The sensor of claim 10, wherein a relative dielectric constant of the intermediate layer is greater than 2.

14. The sensor of claim 10, wherein the first conductive layer of the each of the first sensing structure, the second sensing structure, the third sensing structure, or the fourth sensing structure is a grounding electrode.

15. The sensor of claim 14, wherein two sensing structures on a same side surface of the flexible substrate share the first conductive layer, and the second conductive layers of the two sensing structures on the same side surface are spaced apart in a short axis direction of the flexible substrate.

16. The sensor of claim 14 or 15, wherein a connecting member is provided around a periphery of the sensor, and two ends of the connecting member are connected to the first conductive layers on two side surfaces of the flexible substrate in the thickness direction respectively.

17. The sensor of claim 15, wherein a projection of the first conductive layer of the two sensing structures on the same side surface of the flexible substrate overlies a projection of the second conductive layer of the two sensing structures on the same side surface of the flexible substrate.

18. The sensor of any one of claims 1-6, and 9, wherein each of the first sensing structure, the second sensing structure, the third sensing structure, or the fourth sensing structure includes a second conductive layer, a second intermediate layer, a third conductive layer, a first intermediate layer, and a first conductive layer stacked sequentially away from the flexible substrate in the thickness direction.

19. The sensor of claim 18, wherein two sensing structures on a same side surface of the flexible substrate share the first conductive layer or the second conductive layer, and the third conductive layers of the two sensing structures on the same side surface are spaced apart in a short axis direction of the flexible substrate.

20. The sensor of claim 19, wherein the first conductive layer and the second conductive layer of each of the first sensing structure, the second sensing structure, the third sensing structure, or the fourth sensing structure are both grounding electrodes.

21. The sensor of claim 20, further comprising a connecting member, the connecting member is provided around a periphery of the sensor, and two ends of the connecting member are connected to the first conductive layers on both side surfaces of the flexible substrate in the thickness direction respectively.

22. The sensor of any one of claims 1-6, and 9, wherein each of the first sensing structure, the second sensing structure, the third sensing structure, or the fourth sensing structure includes an intermediate layer and a first conductive layer stacked sequentially away from the flexible substrate in the thickness direction, and the flexible substrate is made of an elastic conductive material.

23. The sensor of claim 22, wherein a conductivity of the flexible substrate is greater than a conductivity of the intermediate layer.

24. The sensor of claim 22, wherein a resistance between two surfaces of the intermediate layer spaced apart in the thickness direction is greater than 0.8 MΩ.

25. The sensor of claim 22, wherein a relative dielectric constant of the intermediate layer is greater than 2.

26. The sensor of claim 22, wherein the first conductive layer of the each of the first sensing structure, the second sensing structure, the third sensing structure, or the fourth sensing structure is a grounding electrode.

27. The sensor of claim 26, wherein the first conductive layers of two sensing structures on a same side surface of the flexible substrate are spaced apart in a short axis direction of the flexible substrate.

28. The sensor of claim 22, wherein the each of the first sensing structure, the second sensing structure, the third sensing structure, or the fourth sensing structure includes a second intermediate layer, a third conductive layer, a first intermediate layer, and the first conductive layer stacked sequentially away from the flexible substrate in the thickness direction.

29. The sensor of claim 28, wherein two sensing structures on a same side surface of the flexible substrate share the first conductive layer, and the third conductive layers of the two sensing structures on the same side surface are spaced apart in a short axis direction of the flexible substrate .

30. The sensor of claim 29, wherein the first conductive layer of the each of the first sensing structure, the second sensing structure, the third sensing structure, or the fourth sensing structure and the flexible substrate are both a grounding electrode.

31. The sensor of claim 29, further comprising a connecting member, the connecting member is provided around the two sensing structures on the same side surface of the flexible substrate, one end of the connecting member is connected to the first conductive layer of the two sensing structures on the same side surface, and the other end of the connecting member is connected to a side surface of the flexible substrate where the two sensing structures are located.

32. The sensor of any one of claims 1-31, wherein the flexible substrate includes a short axis direction and a long axis direction both perpendicular to the thickness direction, and a ratio of a size of the sensor in the short axis direction to a size of the sensor in the thickness direction is in a range of 1:1 to 10:1.

33. The sensor of claim 1, wherein the first parameters are a current value or a voltage value, and the processing circuit determines the deformation in the at least two dimensions of the flexible substrate based on the current value or the voltage value; or
the processing circuit determines a capacitance value or a resistance value of each of the first sensing structure and the second sensing structure based on the current value or the voltage value, and determines the deformation in the at least two dimensions of the flexible substrate based on the capacitance value or the resistance value.

34. The sensor of any one of claims 10-33, wherein the first conductive layer of the each of the first sensing structure, the second sensing structure, the third sensing structure, or the fourth sensing structure is covered with a first protective structure on a side surface away from the flexible substrate.

35. The sensor of claim 34, wherein an exposed surface of the flexible substrate is covered with a second protective structure.

36. A smart glove, comprising:

   a glove body;
   a sensor of any one of claims 1-35; and
   a processor configured to receive and process data collected by the sensor;
   wherein the sensor is located in any one or more regions of the glove body corresponding to finger joints, metacarpophalangeal joints, carpometacarpal joints, and wrist joints of a user.

37. A smart garment, comprising:

a garment body;

a sensor of any one of claims 1-35; and

a processor configured to receive and process data collected by the sensor;

wherein the sensor is located in any one or more regions of the garment body corresponding to shoulder joints, spine joints, hip joints, and ankle joints of a user.

**1**

Flexible substrate 11

First sensing structure 12

Second sensing structure 13

processing circuit 16

**FIG. 1**

**FIG. 2**

**FIG. 3**

S1

A

Z-axis

Y-axis

B

S2

X-axis

12

14

11

15

13

**FIG. 4A**

12

S1

13

S2

14

11

15

**FIG. 4B**

**FIG. 5A**

**FIG. 5B**

**FIG. 6**

**FIG. 7**

**FIG. 8**

**FIG. 9**

**FIG.10**

**FIG. 11**

**FIG. 12**

**FIG. 13**

**FIG. 14**

X-axis

Upper
protective
layer

121-131

12-1

12    123

12-2

122-132

11

142-152

14-2

14    143

14-1

141-151

121-131

13-1

133    13

13-2

122-132

142-152

15-2

153    15

15-1

141-151

Z-axis

17

Lower
protectiv
e layer

**FIG. 15**

X-axis

Z-axis

12 { 121
12-13

131 } 13
12-13

11

14 { 14-15
141

14-15 } 15
151

**FIG. 16**

X-axis

Z-axis

12 { 121-131
12-1
123
12-2

121-131
13-1
133
13-2 } 13

11

14 { 14-2
143
14-1
141-151

15-2
153
15-1
141-151 } 15

**FIG. 17**

X-axis

Z-axis

121-131
12-1
123
12-2
12
11
14-2
143
14-1
141-151
14
121-131
13-1
133
13-2
13
15-2
153
15-1
141-151
15
17

**FIG. 18**

X-axis

Z-axis

111'
112'
11'
121
122
12
142
141
14
131
12-13
132
13
11
152
14-15
151
15
122'
121'
12'

**FIG. 19**

**FIG. 20**

**FIG. 21A**

**FIG. 21B**

4

41

1-7          1-8

Y-axis          Y-axis

1-7

Y-axis          Y-axis

**FIG. 22A**

41

1-10

1-9          1-7

Y-axis

**FIG. 22B**

## INTERNATIONAL SEARCH REPORT

| International application No. |
| --- |
| **PCT/CN2023/124295** |

**A.    CLASSIFICATION OF SUBJECT MATTER**

G01B7/16(2006.01)i

According to International Patent Classification (IPC) or to both national classification and IPC

**B.    FIELDS SEARCHED**

Minimum documentation searched (classification system followed by classification symbols)

IPC:G01B

Documentation searched other than minimum documentation to the extent that such documents are included in the fields searched

Electronic data base consulted during the international search (name of data base and, where practicable, search terms used)

CNABS, CNTXT: 柔性, 变形, 形变, 弯曲, 侧弯, 压缩, 拉伸, 测量, 探测, 检测, 电容, 电阻, 二维, 两个, 维度, 方向, 层, 多层, 差分, 共模, 干扰, 噪声, 手套, 穿戴; VEN, WOTXT, EPTXT, USTXT: flexibility, flexible, deformation, bend, compressible, stretch, measure, detection, capacitance, resistance, two dimension, direction, layer, difference, commom mode, interfere, glove, wearable

**C.    DOCUMENTS CONSIDERED TO BE RELEVANT**

| Category* | Citation of document, with indication, where appropriate, of the relevant passages | Relevant to claim No. |
| --- | --- | --- |
| X | CN 116007488 A (SHANGHAI UDEXREAL INTELLIGENT TECHNOLOGY CO., LTD.) 25 April 2023 (2023-04-25)<br>        description, paragraphs 0002-0261, and figures 1-27 | 1, 4-5, 10-15, 17-20, 22-37 |
| Y | CN 116007488 A (SHANGHAI UDEXREAL INTELLIGENT TECHNOLOGY CO., LTD.) 25 April 2023 (2023-04-25)<br>        description, paragraphs 0002-0261, and figures 1-27 | 2-3, 6-9, 16, 21 |
| Y | CN 215120758 U (SHENZHEN NEW DEGREE TECHNOLOGY CO., LTD.) 10 December 2021 (2021-12-10)<br>        description, paragraphs 0043-0047, and figures 1-7 | 2-3, 6-9, 16, 21 |
| Y | US 8941392 B1 (BEND LABS, INC.) 27 January 2015 (2015-01-27)<br>        description, columns 3-15, and figures 1A-9C | 2-3, 6-9, 16, 21 |
| A | CN 115112272 A (NATIONAL UNIVERSITY OF DEFENSE TECHNOLOGY OF PLA) 27 September 2022 (2022-09-27)<br>        entire document | 1-37 |

☐ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **29 May 2024** | **13 June 2024** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2023/124295**

| Patent document cited in search report | | | Publication date (day/month/year) | Patent family member(s) | | | Publication date (day/month/year) |
|---|---|---|---|---|---|---|---|
| CN | 116007488 | A | 25 April 2023 | CN | 219178484 | U | 13 June 2023 |
| CN | 215120758 | U | 10 December 2021 | None | | | |
| US | 8941392 | B1 | 27 January 2015 | US | 2015048849 | A1 | 19 February 2015 |
| | | | | EP | 3033587 | A1 | 22 June 2016 |
| | | | | EP | 3033587 | B1 | 21 July 2021 |
| | | | | US | 2015054527 | A1 | 26 February 2015 |
| | | | | US | 9222764 | B2 | 29 December 2015 |
| | | | | US | 2017074637 | A1 | 16 March 2017 |
| | | | | US | 9874431 | B2 | 23 January 2018 |
| | | | | US | 2016033255 | A1 | 04 February 2016 |
| | | | | US | 9476692 | B2 | 25 October 2016 |
| | | | | WO | 2015026738 | A1 | 26 February 2015 |
| | | | | CN | 105683703 | A | 15 June 2016 |
| | | | | CN | 105683703 | B | 14 November 2017 |
| CN | 115112272 | A | 27 September 2022 | None | | | |

Form PCT/ISA/210 (patent family annex) (July 2022)